# EUROPÄISCHE PATENTSCHRIFT

(11) **EP 2 410 928 B1**
(45) Veröffentlichungstag und Bekanntmachung des Hinweises auf die Patenterteilung: **03.08.2016**
(21) Anmeldenummer: 10711015.7
(22) Anmeldetag: 25.03.2010
(51) Int. Cl.: A61B 90/00

(54) **SET ZUR SCHAFFUNG EINES KÜNSTLICHEN MAGENEINGANGES**
KIT FOR PROVIDING AN ARTIFICIAL STOMACH ENTRANCE
KIT POUR LA CRÉATION D'UNE GASTROSTOMIE

(30) Priorität: 25.03.2009 DE 202009004536 U
(43) Veröffentlichungstag der Anmeldung: 01.02.2012
(73) Patentinhaber: Marx, Karl-Heinz, 21244 Buchholz (DE)
(72) Erfinder: Marx, Karl-Heinz, 21244 Buchholz (DE)
(74) Vertreter: Hauck Patentanwaltspartnerschaft mbB
(86) Internationale Anmeldenummer: PCT/EP2010/001875
(87) Internationale Veröffentlichungsnummer: WO 2010/108678

(56) Entgegenhaltungen:
- EP-A1- 0 378 095
- WO-A1-02/26293
- WO-A1-03/088854
- WO-A2-96/33752
- WO-A2-2005/046759
- DE-C1- 19 955 071
- DE-U1-202004 008 827
- GB-A- 2 425 483
- US-A- 4 191 191
- US-A- 4 393 873
- US-A- 5 009 639
- US-A- 5 308 325
- US-A- 5 312 362
- US-A- 5 366 446
- US-A- 5 549 594
- US-A- 5 683 416
- US-A- 6 077 243
- US-A1- 2003 109 830
- US-A1- 2004 039 348
- US-A1- 2004 106 899
- US-A1- 2007 184 717
- US-A1- 2008 021 385
- US-A1- 2008 058 721
- US-A1- 2008 140 002

## Beschreibung

Die Erfindung bezieht sich auf ein Set zur Schaffung eines künstlichen Mageneinganges.

Implantate bzw. PEG-Sonden zur Verwerdung bei der perkutanen Nahrungszufuhr werden bei der intragastralen Lang- sowie Kurzzeiternährung beim Patienten eingesetzt. Hierbei handelt es sich um eine Ernährungsart, die immer dann zweckmäßig ist, wenn die Nahrungsaufnahme über Mund und Speiseröhre nicht mehr funktioniert. Gegenüber der venösen Ernährung hat die perkutane Nahrungszufuhr den Vorteil, dass der Verdauungstrakt mit Nahrungsmitteln belastet wird.

Für die perkutane Nahrungszufuhr wird eine PEG-Sonde in einen durch die Bauchdecke zum Magen führenden Stichkanal eingesetzt und dann durch das Implantat flüssige Nahrung zugeführt.

Zum Setzen der PEG-Sonde wird zur Zeit in etwa 90 % der Fälle die Fadendurchzugsmethode nach Keimling eingesetzt. Bei dieser Methode wird durch die Speiseröhre ein Gastroskop eingeführt, mit dem der Magen durch Luftinsufflation gefüllt und somit dicht an die Bauchdecke angedrückt wird. Ein Lichtpunkt in einer Austrittsöffnung des Gastroskopes kann in einem dunklen Raum durch Magen- und Bauchdecke hindurch wahrgenommen werden (Diaphanoskopie). Eine Kanüle bzw. ein Tubus mit einer Nadel wird auf den Lichtpunkt angesetzt und die Nadel eingestochen. Durch die Kanüle hindurch wird ein Faden eingeschoben. Das Ganze wird über einen Videomonitor überwacht. Das Bild aus dem Mageninneren wird über ein Videogastroskop, welches an einen Videoprozessor angeschlossen ist und dieser wiederum an einen Monitor, nach außen übertragen. Eine Fasszange wird durch das Gastroskop eingeführt und der Faden durch das Gastroskop aus dem Mund des Patienten herausgezogen. Danach wird das Gastroskop entnommen. Wenn das Gastroskop nicht mehr liegt, wird der Faden mit der PEG-Sonde durch den Mund des Patienten in den Magen hereingezogen und die PEG-Sonde wird an einem Ansatzstück aus der Bauchdecke herausgezogen. Schließlich wird von außen eine Abdeckung bzw. ein Verschluss aufgesetzt.

Bei der Fadendurchzugsmethode ist zum einen der hohe Aufwand nachteilig. Ferner kann das Hindurchführen der PEG-Sonde durch die unsterilen Bereiche des Mundes und das anschließende Einsetzen in die offene Wunde im Bauchdeckenbereich zu Infektionen führen. In der Literatur wird als häufigste postoperative Komplikation die peristomale Wundinfektion angegeben, die trotz prophylaktischer Antiobiotikagabe in bis zu 30 % der Fälle eintritt, in denen mittels der Fadendurchzugsmethode gearbeitet wird. Zur Kontrolle der Lage der PEG-Sonde kann zudem eine zweite Gastroskopie notwendig sein, wobei die Patientenbelastung durch die Gastroskopien sehr hoch ist.

Nachdem der Stichkanal ausgeheilt ist, kann die PEG-Sonde durch einen sogenannten "Button" ersetzt werden. Für die Entnahme der PEG-Sonde muss wiederum das Gastroskop eingesetzt und die Fasszange eingeführt werden. Die PEG-Sonde kann dann mittels der Fasszange von der Innenseite des Magens aus herausgezogen werden. Da die Fadendurchzugsmethode nur in der Klinik oder in Praxen durchgeführt werden kann, in denen endoskopiert wird, müssen Patienten dorthin verlegt werden, was die Anwendung ebenfalls aufwendig macht.

Bei einer weiter entwickelten Methode wird bei der Diaphanoskopie ein Trokar mit einer Trokarhülse von außen an die Bauchdecke angesetzt und eingestochen. Durch die Trokarhülse hindurch wird eine PEG-Sonde eingeführt. Das Einführen der PEG-Sonde wird vom Magen aus durch ein Gastroskop optisch kontrolliert. Wenn die Sonde eingeführt ist, wird ein Ballon am Ende der Sonde aufgeblasen bzw. mit Flüssigkeit gefüllt. Danach wird die in Längsrichtung teilbare Kanüle (Splitting-Kanüle) auseinandergezogen bzw. auseinandergeteilt und entfernt, sodass die PEG-Sonde liegt. Schließlich wird ein auf der PEG-Sonde angebrachter Abschluss auf die Bauchdecke abgesenkt und ein Versorgungsschlauch auf den Abschluss aufgesetzt. Diese Methode betrifft nur die PEG-Sonde (Ersteinlage). Der Button (Zweiteinlage) wird nach Ausheilen des Stichkanals in der bisherigen Weise gesetzt.

PEG-Sonden und Buttons der vorbeschriebenen Art sind in der EP 0 667 763 B1 beschrieben.

Die US 2008/058721 A1 beschreibt ein Set zur Schaffung eines künstlichen Mageneinganges, umfassend einen Trokar mit einer Trokarhülse zum Einstechen in den Magen und eine nach Entnahme des Trokars aus der Hülse in diese einführbare PEG-Sonde, wobei der Trokar eine Trokarspitze aufweist.

Bei den bekannten Trokaren mit Trokarhülse ist das Einstechen in die Bauchdecke und Magen sehr mühselig. Es besteht die Gefahr, dass beim Einstechen die gegenüberliegende Magenwand verletzt wird. Des Weiteren schiebt der Trokar den Magen weg.

Davon ausgehend liegt der Erfindung die Aufgabe zugrunde, ein anwendungsfreundlicheres Set zur Schaffung eines künstlichen Mageneinganges zur Verfügung zu stellen.

Die Aufgabe wird durch das Set mit den Merkmalen des Anspruchs 1 gelöst.

Gemäß Anspruch 1 umfasst das erfindungsgemäße Set zur Schaffung eines künstlichen Mageneinganges einen Trokar mit einer Trokarhülse zum Einstechen durch die Bauchdecke in den Magen und eine nach Entnahme des Trokars aus der Trokarhülse in diese einführbare PEG-Sonde, wobei der Trokar eine Trokarspitze aufweist, die eine Konusspitze ist, bei der der halbe Konuswinkel 5° bis 12 ° beträgt und der Trokar einen Durchmesser von 4 bis 8 mm aufweist.

Das erfindungsgemäße Set hat einen Trokar mit einer in bislang nicht bekannter Weise spitz zulaufenden Trokarspitze. Diese hat einen Spitzenwinkel von maximal 5 bis 12°. Bei dem Spitzenwinkel handelt es sich um den Winkel, der zwischen einer Mantellinie der Trokarspitze und ihrer Mittelachse gegeben ist. Die Trokarspitze ist z.B. als Mehrkantspitze und/oder als Konusspitze ausgeführt. Bei der Mehrkantspitze ist der Spitzenwinkel der Anschliffwinkel und bei der Konusspitze ist der Spitzenwinkel der halbe Konuswinkel. Die besonders spitz zulaufende Trokarspitze begtinstigt ein allmähliches Durchdringen von Bauchdecke und Magenwand. Auf ein Andrücken des Magens von innen mittels Gastroskop oder ein Annähen des Magens an die Bauchdecke kann verzichtet werden. Es reicht aus, den Magen über das Gastroskop mit Luft aufzublähen, sodass er von innen an die Bauchdecke angedrückt wird. Das Gastroskop zum Einbringen von Luft in den Magen kann zusätzlich zum Beobachten des Durchstoßvorganges verwendet werden. Demgegenüber dringen die bekannten Trokare mit verhältnismäßig stumpfer Spitze erst bei Anlage hoher Druckkraft in die Bauchdecke ein, drücken die Magenwand vor sich her und durchstoßen sie schlagartig. Gemäß einer Ausgestaltung hat die Trokarspitze eine Länge von etwa 8 mm bis 22 mm. Für Kinder beträgt die Länge vorzugsweise bis zu etwa 12 mm. Für Erwachsene beträgt sie vorzugsweise bis zu etwa 20 mm. Durch die Längenbegrenzung sollen Verletzungen beim Einschieben in den Magen vermieden werden. Der Trokar weist einen Durchmesser im Bereich von etwa 4 bis 8 mm auf. Für Kinder wird eher ein geringerer Durchmesser von bis zu etwa 4 mm gewählt. Für Erwachsene kann der Durchmesser auch größer sein, und zwar bis zu etwa 8 mm. Bevorzugt ist die gesamte Trokarspitze konisch. Die Trokarspitzen kann insbesondere von einem einzigen Konus gebildet sein oder von verschiedenen konischen Abschnitten. Der distale konischen Abschnitt kann insbesondere eine Bohrspitze sein. Bei einer grundsätzlich konischen Bohrspitze kann aber auch distal eine Mehrkantspitze als Bohrspitze vorhanden sein.

Gemäß einer Ausgestaltung ist der Trokar scharfkantig angeschliffen. Hierdurch kann das allmähliche Eindringen und. Durchdringen von : Bauchdecke und Magenwand weiter gefördert werden.

Gemäß einer weiteren Ausgestaltung hat der Trokar eine Dreikantspitze.

Gemäß einer weiteren Ausgestaltung ist die Konusspitze mit einem Außengewinde versehen. Aufgrund des Außengewindes kann das allmähliche Eindringen durch eine Schraubbewegung des Trokars unterstützt werden. Die Konusspitze kann korkenzieherartig ausgeführt sein.

Gemäß einer weiteren Ausgestaltung weist die Trokarspitze distal eine Bohrspitze auf. Die Bohrspitze dient dem Anbohren eines Loches in der Bauchdecke. Bevorzugt hat sie hierfür einen größeren Spitzenwinkel als die Trokarspitze. Bevorzugt beträgt der Spitzenwinkel der Bohrspitze 20°. Weiterhin bevorzugt beträgt er über 30°. Die Bohrspitze kann insbesondere als Mehrkantspitze oder als Konusspitze ausgeführt sein.

Gemäß einer Ausgestaltung umfasst das Set eine PEG-Sonde mit einer in den Magen einsetzbaren, distalen Erweiterung in Form eines Ballons aus einem flexiblen Material, das nicht dehnbar ist oder allenfalls eine geringe Dehnbarkeit aufweist, die geringer als die Dehnbarkeit von Silikon ist, die distal mindestens eine Öffnung aufweist und einen proximal daran anschließenden rohrförmigen Bereich, wobei ein Ernährungskanal von einer proximalen Emährungsöffnung des rohrförmigen Bereiches aus durch den rohrförmigen Bereich bis zu der mindestens einen Öffnung erstreckt ist und der Ballon mit einem Füllkanal verbunden ist, der sich entlang des rohrförmigen Bereichs bis zum proximalen Ende erstreckt und eine Einfüllöffnung mit einem Verschluss aufweist, wobei der Ballon im kollabierten Zustand am rohrförmigen Bereich anliegt und in diese Zustand durch Sicherungsmittel auf dem rohrförmigen Bereich gesichert ist.

Bei herkömmlichen PEG-Sonden besteht der Ballon aus einem elastischen Material. Beim Füllen des Ballons mit einem Fluid wird das Material des Ballons beträchtlich gedehnt. Zudem steht der Ballon, der sich an der Innenwand des Magens abstützt, ständig unter Spannung. Dies kann dazu führen, dass der Ballon platzt und die PEG-Sonde nicht mehr an der Innenseite der Magenwand abstützt. Erfindungsgemäß kommt hingegen der Ballon aus einem flexiblen Material zum Einsatz, das nicht dehnbar ist oder allenfalls eine geringe Dehnbarkeit aufweist. Der Vorteil dieses Ballons ist, dass er nach dem Füllen mit einem Fluid unter geringerer Spannung als ein herkömmlicher Ballon steht. Infolgedessen wird der Ballon der PEG-Sonde des erfindungsgemäßen Sets weniger leicht beschädigt.

Das Material des Ballons hat eine geringere Dehnbarkeit als Silikon, das bei herkömmlichen PEG-Sonden zum Einsatz kommt. Gemäß einer bevorzugten Ausgestaltung kommt als Material für den Ballon PUR (Polyurethan) oder ein anderes flexibles Material mit einer ähnlich geringen Dehnbarkeit zum Einsatz. Bevorzugt kommt ein Kunststoff mit einer ähnlich geringen Dehnbarkeit zum Einsatz.

Bei einer herkömmlichen PEG-Sonde kann sich der elastische Ballon im Wesentlichen glatt an die PEG-Sonde anlegen. Bei der PEG-Sonde des erfindungsgemäßen Sets ist dies grundsätzlich nicht der Fall, da sich der Ballon nicht elastisch zusammenzieht. Der Ballon liegt im kollabierten Zustand am rohrförmigen Bereich der PEG-Sonde an und ist in diesem Zustand durch Sicherungsmittel auf dem rohrförmigen Bereich gesichert. Das Sicherungsmittel verhindert, dass sich der Ballon vor dem Einschieben durch die Trokarhülse in den Magen öffnet. Das Sicherungsmittel schützt den Ballon beim Einführen in die Trokarhülse vor Beschädigung. Das Sicherungsmittel kann beispielsweise ein auf den Ballon aufgebrachtes festes Medium sein, das sich im Magen des Patienten auflöst, insbesondere durch Einwirkung der Magensäure. Aus dem Bereich der Arzneimittel sind beispielsweise Gele bekannt, die außerhalb des Magens fest sind und sich im Milieu des Magens auflösen sowie biologisch vertiäglich sind. Derartige Gele können beispielsweise als Sicherungsmittel zum Einsatz kommen. Das Medium kann einen antiseptischen Wirkstoff enthalten. Das Sicherungsmittel können aber auch von einer Einführhülse gebildet sein, in die die PEG-Sonde vor dem Einsetzen in die Trokarhülse eingesetzt ist. Mit Hilfe der Einführhülse wird die PEG-Sonde in die Trokarhülse eingesetzt und danach kann die Einführhülse von der PEG-Sonde abgezogen werden. Dies kann geschehen, bevor oder nachdem die PEG-Sonde mit dem kollabierten Ballon aus dem distalen Ende der Trokarhülse herausgeschoben wird.

Gemäß einer weiteren Ausgestaltung umfasst das Set eine Trokarhülse die proximal ein Ventil aufweist, durch das der Trokar in die Trokarhülse einführbar ist und das nach Entnahme des Trokars aus der Trokarhülse luftdicht abschließt, wo bei das Ventil mindestens eine Schwächungslinie zum mindestens teilweisen Auftrennen des Ventils aufweist, deren Auftrennen das Entfernen des Ventils von der Trokarhülse erleichtert.

Bei dem erfindungsgemäßen Set ist der Trokar mit der Trokarhülse in die Bauchdecke und die Magenwand einstechbar. Hierbei kann, durch Einleiten von Luft in den Magen sichergestellt werden, dass der Magen innen an der Bauchdecke anliegt. Bei Entnahme des Trokars aus der Trokarhülse kann bei herkömmlichen Sets Überdruck aus dem Magen entweichen. Infolgedessen kann sich der Magen von der Bauchdecke entfernen und von der Trokarhülse abrutschen, sodass die korrekte Platzierung der PEG-Sonde mit dem distalen Ende im Magen nicht gewährleistet ist. Bei dem erfindungsgemäßen Set wird durch das Ventil die proximale Öffnung der Trokarhülse abgedichtet, wenn der Trokar entnommen ist. Infolgedessen bleibt der Überdruck im Magen erhalten und die Magenwand rutscht nicht von der Trokarhülse ab. Das Setzen der PEG-Sonde wird folglich durch das erfindungsgemäße Set unterstützt und vereinfacht.

Durch das luftdichte Abschließen ist zunächst gewährleistet, dass der Luftdruck im Magen bis zum Einführen der PEG-Sonde nicht so sehr abfällt, dass sich die Magenwand in erheblichem Umfang von der Bauchdecke entfernt und von der Trokarhülse abrutscht. Eine absolute Luftdichtigkeit ist grundsätzlich nicht erforderlich. Angestrebt ist jedoch, das Ventil so auszuführen, dass es nach dem Herausziehen des Trokars möglichst Luftdicht ist.

Grundsätzlich kann das Ventil unlösbar mit der Trokarhülse verbunden sein. Gemäß einer bevorzugten Ausgestaltung weisen jedoch die Trokarhülse und das Ventil Haltemittel zum lösbaren Halten des Ventils an der Trokarhülse auf. Diese Ausgestaltung begünstigt insbesondere eine getrennte Fertigung von Trokarhülse und Ventil, da das Ventil nachträglich an der Trokarhülse montierbar ist. Ferner kann bei dieser Ausgestaltung das Ventil nach Gebrauch demontierbar und gegen ein unbenutztes Ventil austauschbar sein. Dies ist insbesondere zweckmäßig, wenn eine wieder verwendbare Trokarhülse zum Einsatz kommt. Die Lösbarkeit des Ventils kann aber auch vorteilhaft für das Entfernen der Trokarhülse aus dem Einsteckkanal sein. Dies ist insbesondere der Fall, wenn die Trokarhülse in Längsrichtung in zwei Hälften zerlegbar ist, um sie von einer PEG-Sonde mit einer proximalen Verbreiterung abzunehmen. Das Ventil kann zuvor abgenommen werden, was das Zerlegen der Trokarhülse in zwei Hälften erleichtert.

Gemäß einer weiteren Ausgestaltung haben das Ventil und die Trokarhülse Schnappmittel, zum Aufschnappen des Ventils auf die Trokarhülse. Infolgedessen ist das Ventil besonders einfach auf die Trokarhülse montierbar und von dieser abnehmbar.

Das Ventil kann in verschiedener Weise ausgestaltet sein. Gemäß einer Ausgestaltung umfasst es eine Abdeckscheibe aus elastischem Material oder aus einem Material mit einem Memoryeffekt, die randseitig an der Trokarhülse festgelegt ist und den Durchgangskanal der Trokarhülse versperrt und durch die der Trokar zum Einführen in den Durchgangskanal einsteckbar ist, wobei die Einstechstelle in der Abdeckscheibe nach Herausziehen des Trokars aufgrund elastischer Rückstellkräfte oder des Memoryeffektes schließt: Die Abdeckscheibe legt sich beim Einstechen des Trokars elastisch oder aufgrund des Memoryeffektes an den Umfang des Trokars an und die Einstechstelle schließt sich selbsttätig, wenn der Trokar herausgezogen wird. Dadurch wird eine hinreichende Abdichtung der Trokarhülse erreicht, so dass der Uberdruck im Magen bis zum Einsetzen der PEG-Sonde nicht in einem abträglichen Ausmaß abfällt.

Gemäß einer weiteren Ausgestaltung hat die Abdeckscheibe einen nach außen umgebogenen, umlaufenden Rand zum Aufschnappen auf einen umlaufenden Wulst am proximalen Ende der Trokarhülse. Bei dieser Ausgestaltung kann die Abdeckscheibe als Wegwerfteil ("Disposable") ausgeführt sein, wogegen der Trokar und/oder die Trokarhülse ein wiederverwendbares Bauteil sein können.

Die Abdeckscheibe kann ursprünglich geschlossen sein und von dem eindringenden Trokar eingeschnitten und geöffnet werden. Gemäß einer weiteren Ausgestaltung weist die Abdeckscheibe von vornherein mindestens einen Schlitz zum Einstechen des Trokars auf. Hierdurch wird ein kontrolliertes Öffnen der Abdeckscheibe ermöglicht, das eine wiederholte Abdichtung nach Entnahme des Trokars begünstigt. Bevorzugt entspricht die Anzahl der Schlitze in der Abdeckwand der Anzahl der Kanten der Mehrkantspitze des Trokars, wobei die Schlitze von einer zentralen Schnittstelle ausgehend entsprechend der Ausrichtung der Mehrkantspitze ausgerichtet sind. Der Trokar kann mit der Mehrkantspitze auf die Schlitze ausgerichtet werden, wodurch ein besonders kontrolliertes Öffnen des Ventiles erreicht wird.

Das Ventil weist mindestens eine Schwächungslinie zum zumindest teilweisen Auftrennen des Ventils auf. Nach zumindest teilweisem Auftrennen kann das Ventil leichter von der Trokarhülse entfernt werden. Die Schwächungslinie ist beispielsweise eine Linie, entlang der die Wandstärke des Ventils reduziert ist oder eine Perforationslinie. Gemäß einer weiteren Ausgestaltung weist das Ventil randseitig eine Aufreißlasche auf. Die Aufreißlasche erleichtert das anfängliche Aufreißen des Ventils entlang der Schwächungslinie. Gemäß einer weiteren Ausgestaltung verläuft die Schwächungslinie in Radialrichtung und/oder in Umfangsrichtung der Abdeckscheibe. Wenn sich die Schwächungslinie in Radialrichtung erstreckt, kann die Abdeckscheibe in Segmente aufgetrennt werden. Falls die Abdeckscheibe einen umlaufenden Rand hat, erstreckt sich bevorzugt die Schwächungslinie ein Axialrichtung zusätzlich über den umfänglichen Rand, damit die Abdeckscheibe ausgehend vom Rand aufgerissen werden kann. Insbesondere bei einer Abdeckscheibe mit umlaufendem Rand kann die Schwächungslinie vorteilhaft in Umfangsrichtung der Abdeckscheibe verlaufen, um ein zumindest teilweises Abziehen des Randes entlang der Schwächungslinie zu ermöglichen.

Das Ventil bzw. die Abdeckscheibe ist beispielsweise aus Silikon oder Gummi oder TPE (thermoplastisches Elastomer) hergestellt.

Gemäß einer weiteren Ausgestaltung umfasst das Set eine Trokarhülse, die in einem Abstand vom distalen Ende am Umfang eine Markierung aufweist, zum Anzeigen, ob die Trokarhülse tief genug in Bauchdecke und Magen eingeschoben ist.

Bei dem erfindungsgemäßen Set wird der Trokar mit der Trokarhülse in der bereits beschriebenen Weise in Bauchdecke und Magenwand eingestochen. Bei herkömmlichen Sets ist das Ausmaß des Einstechens der Erfahrung des Operateurs überlassen. Dies konnte zu einem unzureichenden Eindringen von Trokar und Trokarhülse führen, mit der Gefahr, dass die Magenwand von der Trokarhülse abrutscht und die PEG-Sonde falsch gelegt wird. Mit dem erfindungsgemäßen Set ist mit Hilfe der Markierung eine korrekte Eindringtiefe der Trokarhülse in Bauchdecke und Magenwand gewährleistet. Der Abstand der Markierung vom distalen Ende der Trokarhülse basiert auf Erfahrungswerten. Ggfs. können verschiedene Markierungen angebracht werden, die eine optimale Position in Abhängigkeit von Alter, Geschlecht, Körpergewicht etc. angeben.

Gemäß einer Ausgestaltung ist die Markierung in einem Abstand von 2 bis 5 cm vom distalen Ende der Trokarhülse angeordnet. Dieser Bereich beruht auf

Erfahrungswerten. Bevorzugt ist sie in einem Abstand von etwa 3 cm vom distalen Ende der Trokarhülse angeordnet. Die Markierung kann verschieden ausgeführt sein, beispielsweise als Rille oder kleine Erhabenheit am Umfang der Trokarhülse. Gemäß einer Ausgestaltung ist sie ein Strich, der beispielsweise aufgedruckt oder aufgemalt ist.

Gemäß einer weiteren Ausgestaltung umfasst das Set eine PEG-Sonde aus flexiblem Material, die eine in den Magen einsetzbare, distale Erweiterung hat, die mindestens eine Öffnung aufweist, und einen proximal an die . Erweiterung anschließenden rohrförmigen Bereich, wobei von einer proximalen Ernährungsöffnung des rohrförmigen Bereichs aus durch den rohrförmigen Bereich bis zu der mindestens einen Öffnung der Erweiterung ein Ernährungskanal verläuft, und einen Stab mit einem distalen Ende uhd einer Handhabe am proximalen Ende, der mit dem distalen Ende in den Ernährungskanal einschiebbar ist.

Bei dem erfindungsgemäßen Set wird in der beschriebenen Weise der Trokar mit der Trokarhülse gesetzt und der Trokar aus der gesetzten Trokarhülse entnommen. Zum Setzen der PEG-Sonde wird in diese der Stab eingesteckt und hierdurch die flexible PEG-Sonde stabilisiert. Hierdurch wird das Einschieben der PEG-Sonde in die Trokarhülse und ihre korrekte Plazierung begünstigt. Vorzugsweise ist das distale Ende des Stabes stumpf.

Der Stab kann so ausgeführt bzw. angewandt werden, dass er ausschließlich der Stabilisierung der verformbaren PEG-Sonde beim Einschieben in die Trokarhülse dient. Er kann aber auch so ausgeführt bzw. angewandt werden, dass er die PEG-Sonde beim Einführen streckt. Insbesondere zu diesem Zweck ist gemäß einer Ausgestaltung die PEG-Sonde aus einem elastischen Material hergestellt. Ferner ist gemäß einer Ausgestaltung der Stab mit einem distalen Ende gegen ein Widerlager am distalen Ende der PEG-Sonde verschiebbar. Die Streckung der PEG-Sonde ist mit einer Verringerung ihres Außendurchmessers verbunden und dies ermöglicht den Einsatz eines Trokars und einer Trokarhülse mit geringerem Querschnitt. Hierdurch wird die Traumatisierung des Patienten beim Setzen des künstlichen Mageneingangs verringert.

Gemäß einer Ausgestaltung ist das Widerlager ein Boden am distalen Ende der PEG-Sonde oder ein Absatz im Ernährungskanal, auf den der Stab mit dem distalen Ende aufsetzbar ist. Hierfür ist bevorzugt das distale Ende des Stabes stumpf. Falls das Widerlager ein Boden der PEG-Sonde ist, kann mindestens eine Öffnung des Ernährungskanals proximal des Bodens ausgebildet sein. Der Absatz ist vorzugsweise ein umlaufender Absatz. Er kann insbesondere als Ring ausgebildet sein, der in den Ernährungskanal eingebettet ist. Der Absatz ist bevorzugt am distalen Ende des Ernährungskanals angeordnet. Das Widerlager kann auch ein Stopfen sein, der den Ernährungskanal ganz oder teilweise verschließt und sich im Magen des Patienten auflöst. Gemäß einer anderen Ausgestaltung ist das Widerlager mindestens eine Öse am distalen Ende der PEG-Sonde, in die der Stab mit einem Vorsprung an seinem distalen Ende einsetzbar ist. Die Öse kann beispielsweise an einer flexiblen Lasche ausgebildet sein, die am distalen Ende der PEG-Sonde angeordnet ist. Bevorzugt weist die PEG-Sonde zwei einander diametral gegenüberliegende Ösen an ihrem distalen Ende auf. Dementsprechend ist der Stab bevorzugt mit zwei gabelartigen Vorsprüngen an seinem distalen Ende versehen. Mit seinen Vorsprüngen kann der Stab in die Ösen der Laschen eingreifen, die nach innen über die Öffnung des Ernährungskanals geschwenkt sind. Nach Herausziehen des Stabes aus den Ösen können diese nach außen klappen und die Öffnung des Ernährungskanals freigeben.

Gemäß einer Ausgestaltung weist die PEG-Sonde am proximalen Ende eine Verbreiterung auf. Die Verbreiterung erleichtert das Aufspannen der PEG-Sonde mit Hilfe des Stabes, in dem der Operateur mit einer Hand die Verbreiterung und die Handhabe zusammendrückt.

Gemäß einer weiteren Ausgestaltung weist die PEG-Sonde am proximalen Ende einen Verschluss auf. Der Verschluss dient dem Schließen des Ernährungskanals, wenn keine Nahrung zugeführt wird. Ferner kann der Verschluss eine Verbreiterung am proximalen Ende bilden, die das Setzen der PEG-Sonde im gestreckten Zustand mit Hilfe des Stabes erleichtert.

Gemäß einer weiteren Ausgestaltung weist die PEG-Sonde am distalen Ende einen Ballon und einen damit verbundenen und entlang des rohrförmigen Bereichs bis zum proximalen Ende erstreckten Füllkanal mit einem Verschluss an einer proximalen Einfüllöffnung auf, wobei durch den Füllkanal ein Fluid zum Aufpumpen des Ballons einfüllbar ist. Der Ballon bildet die distale Erweiterung. Durch Einfüllen eines Fluids, d.h. einer Flüssigkeit oder eines Gases, kann er nach dem Setzen der PEG-Sonde aufgeweitet werden, um diese an der Innenseite der Magenwand zu halten.

Gemäß einer weiteren Ausgestaltung umfasst das Set eine PEG-Sonde mit einer in den Magen einsetzbaren, distalen Erweiterung in Form eines Ballons, die mindestens eine Öffnung aufweist und einem proximal daran anschließenden rohrförmigen Bereich, wobei ein Ernährungskanal von einer proximalen Ernährungsöffnung des rohrförmigen Bereichs aus durch den rohrförmigen Bereich bis zu der mindestens einen Öffnung erstreckt ist und der Ballon mit einem Füllkanal verbunden ist, der sich entlang des rohrförmigen Bereichs bis zum proximalen Ende erstreckt und eine Einfüllöffnung mit einem Verschluss aufweist, wobei der Verschluss ein den Querschnitt der Einfüllöffnung versperrendes, entfernbares Schließelement aufweist, das Schließelement sowie die PEG-Sonde am proximalen Ende Mittel zum Verriegeln des Schließelementes an der PEG-Sonde aufweisen und das Schließelement ein Mittel zum Ansetzen eines Schlüssels zum Zwecke der Aufhebung der Verriegelung des Schließelementes mit der PEG-Sonde aufweist.

Bei dem erfindungsgemäßen Set wird der Trokar mit der Trokarhülse und die PEG-Sonde in der bereits beschriebenen Weise gesetzt. Zum Verankern der PEG-Sonde an der Innenwand der Bauchdecke wird durch die Einfüllöffnung ein Fluid eingefüllt. Schließlich wird die Einfüllöffnung durch das Schließelement versperrt, das mit der PEG-Sonde verriegelt wird. Zum Entriegeln des Schließelements ist der Einsatz eines Schlüssels erfbrderlich, das an die Mittel zum Ansetzen des Schließelementes angesetzt werden muss. Hierbei kann es sich um einen herkömmlichen Schlüssel handeln, der in ein mit dem Schließelement integriertes Schloss einsetzbar ist und mit den Mitteln zum Verriegeln zusammenwirkt. Gemäß einer bevorzugten Ausgestaltung sind die Mittel zum Ansetzen eines Schlüssels ein Werkzeugangriff zum Ansetzen eines Werkzeuges und ist der Schlüssel von einem Werkzeug gebildet. Bei dem Werkzeug handelt es sich bevorzugt um ein Spezialwerkzeug. Die Notwendigkeit des Einsatzes eines Schlüssels verhindert, dass unbeabsichtigt das Fluid aus dem Ballon abgelassen wird und die PEG-Sonde aus dem Magen herausrutscht. Bei dem Set ist folglich die korrekte Positionierung der PEG-Sonde durch das Schließelement gesichert. Infolgedessen kann es nicht zu Gefahrensituationen kommen, wie beispielsweise die Einspeisung von Flüssignahrung zwischen Bauchdecke und Magenwand, die bereits zu Todesfällen geführt hat.

Gemäß einer Ausgestaltung ist das Verriegelungselement ein Gewindestift, der in ein Innengewinde der Füllöffnung einschraubbar ist.

Gemäß einer weiteren Ausgestaltung ist der Werkzeugangriff ein Werkzeugangriff für einen Schraubenschlüssel, einen Schraubendreher, einen Innenmehrkantschlüssel oder ein anderes Drehwerkzeug. Der Werkzeugangriff ist beispielsweise ein Schlitz, ein Kreuzschlitz, Torx oder Inbus.

Gemäß einer weiteren Ausgestaltung weist der Werkzeugangriff mindestens einen Schlitz, Innenmehrzahn oder Innenmehrkant auf.

Gemäß einer weiteren Ausgestaltung umfasst das Set eine PEG-Sonde mit einer in den Magen einsetzbaren distalen Erweiterung, die distal mindestens eine Öffnung aufweist, und einen proximal daran anschließenden rohrförmigen Bereich, wobei ein Ernährungskanal von einer proximalen Ernährungsöffnung des rohrförmigen Bereichs aus durch den rohrförmigen Bereich bis zu der mindestens einen Öffnung erstreckt ist, wobei die PEG-Sonde in einem Abstand von der distalen Erweiterung und/oder dem proximalen Ende eine Markierung aufweist, zum Anzeigen, ob die PEG-Sonde tief genug in die Trokarhülse eingeschoben ist.

Die Markierung in einem Abstand von der distalen Erweiterung und/oder vom proximalen Ende erleichtert das korrekte Einsetzen der PEG-Sonde in die Trokarhülse. Insbesondere kann bei Anordnung der Markierung in einem geringen Abstand von der distalen Erweiterung durch ein Gastroskop beobachtet werden, dass die PEG-Sonde tief genug eingeschoben ist. Ohne eine Markierung ist es gegebenenfalls schwer möglich, die distale Erweiterung zu erkennen, insbesondere wenn diese als Ballon ausgeführt ist, der eng an der PEG-Sonde anliegt. Diese Markierung kann beispielsweise etwa 1 bis 2 cm proximal von der distalen Erweiterung angeordnet sein. Wenn die Trokarhülse korrekt angebracht ist, ist es grundsätzlich auch möglich, die korrekte Positionierung der PEG-Sonde außen mit Hilfe einer Markierung zu erkennen, die in einem geringen Abstand vom proximalen Ende angeordnet ist.

Gemäß einer bevorzugten Ausgestaltung weist die PEG-Sonde eine distale Erweiterung in Form eines Ballons auf.

Gemäß einer weiteren Ausgestaltung umfasst das Set eine PEG-Sonde mit einer in den Magen einsetzbaren, distalen Erweiterung in Form eines elastischen Ballons mit einer den Ballon umgebenden Einführhülse, in der der Ballon radial elastisch zusammengepresst angeordnet ist, wobei die Einführhülse mit der darin angeordneten PEG-Sonde in die Trokarhülse einführbar ist und die PEG-Sonde mit dem Ballon in distaler Richtung aus der Einführhülse heraus und aus der Trokarhülse herausschiebbar ist, so dass der Ballon expandiert.

Die Einführhülse dient dazu, den elastischen Ballon in Radialrichtung zusammenzupressen, so dass die PEG-Sonde in der Einführhülse in die Trokarhülse einschiebbar ist. Zudem schützt die Einführhülse den Ballon beim Verschieben in der Trokarhülse. Wenn die PEG-Sonde mit dem Ballon aus der Einführhülse herausgeschoben wird, expandiert der Ballon. Sofern dies in der Trokarhülse geschieht, legt er sich an die Innenwand der Trokarhülse an. Danach kann die PEG-Sonde weiter eingeschoben werden, bis der Ballon im Magen vollständig expandiert. Es ist aber auch möglich, die PEG-Sonde innerhalb der Einführhülse mit dem Ballon bis in den Magen einzuschieben und die Einführhülse erst dann zurückzuziehen, so dass der Ballon sogleich im Magen vollständig expandiert. Der Ballon kann insgesamt aus einem elastischen Material bestehen. Es ist aber auch möglich, die PEG-Sonde mit einem Ballon auszuführen, der eine flexible oder elastische Wand hat und dauerhaft mit einem elastischen Medium gefüllt ist. Das elastische Material, aus dem der Ballon gebildet oder mit dem der Ballon gefüllt ist, kann beispielsweise ein elastischer Schaumstoff sein.

Bei dieser Ausgestaltung kann die PEG-Sonde in der Einführhülse vormontiert und in die Trokarhülse eingesetzt werden. Die PEG-Sonde, vor allem der Ballon, kann somit auf einen dünneren Außendurchmesser gebracht werden und zusätzlich wird der Ballon mit der PEG-Sonde beim Einführen durch die Einführhülse geschützt.

Gemäß den Ausführungen des Ballons aus elastischem Material bekommt die PEG-Sonde einen sehr guten, sicheren Halt an der Magenwand. Dieses ist für eine Langzeiternährung sehr gut. Diese PEG-Sonde ist aber nur über den Magen und die Speiseröhre zu entfernen.

Bei Ausführungen der PEG-Sonde aus einem elastischen Material bzw. mit einer Füllung aus elastischem Material kann zusätzlich durch einen Füllkanal in den Ballon ein Kleber oder ein anderes Stabilisierungsmittel eingebracht werden. Das stabilisierende Material dient dazu, die Elastizität des Ballons zu verringern. Hierbei kann es sich um einen aushärtenden Kleber handeln, beispielsweise um einen Fibrinkleber.

Gemäß einer Ausgestaltung kann der Ballon auf der PEG-Sonde mit einer Substanz gefüllt werden, deren Konsistenz veränderbar ist, beispielsweise durch Einflüsse wie Temperatur oder ähnliches. Somit kann die Substanz sehr dünnflüssig in den Ballon gegeben werden und über den Ernährungskanal der PEG-Sonde insofern manipuliert werden, dass die Substanz im Ballon dickflüssiger wird und somit einen größeren Widerstand bildet, wenn der Ballon an die Magenwand herangezogen wird. Beim Herausziehen der PEG-Sonde kann diese Substanz wieder dünnflüssiger gemacht werden, damit sie aus dem Ballon abgesaugt und die PEG-Sonde entfernt werden kann.

Gemäß einer bevorzugten Ausgestaltung ist die Einführhülse eine in Längsrichtung teilbare Hülse, um die Einführhülse nach dem Herausschieben des Ballons aus der Trokarhülse zu entfernen. Die Teilbarkeit der Einführhülse ermöglicht insbesondere, die Einführhülse abzuziehen, wenn die PEG-Sonde am proximalen Ende eine Verbreiterung hat.

Gemäß einer bevorzugten Ausgestaltung besteht die Einführhülse aus einem Kunststoff.

Gemäß einer Weiteren bevorzugten Ausgestaltung weist die Einführhülse am proximalen Ende eine Handhabe auf. Die Handhabe erleichtert das Einschieben der Einführhülse in der Trokarhülse.

Bei einem Set mit einer PEG-Sonde, die einen Ballon aufweist, der über einen Füllkanal befüllbar ist, kann gemäß einer Ausgestaltung in den Füllkanal ein flüssiges Medium eingebracht werden, das im Ballon über Körperwärme oder eine andere Aktivierung (z.B. durch Strahlung) in einen festen oder elastischen Zustand gebracht werden kann. Dies ermöglicht es, die PEG-Sonde sicher an der Innenwand des Magens zu fixieren. Zum Entfernen der PEG-Sonde wird bevorzugt ein Medium eingesetzt, das in seinen flüssigen Ursprungszustand zurückversetzt werden kann, beispielsweise durch gezieltes Kühlen oder Deaktivierung mittels einer anderen Strahlung.

Gemäß einer Ausgestaltung umfasst das Set ein aushärtbares Medium zum Auffüllen eines Ballons der PEG-Sonde. Das Medium ist bevorzugt beim Füllen des

Ballons flüssig und wird danach durch Aktivieren im Ballon ausgehärtet z.B. durch Strahlungs- oder Wärmeeinwirkung. Weiterhin bevorzugt hat es die Eigenschaft, aus dem ausgehärteten Zustand wieder in den flüssigen Zustand überführt werden zu können, z.B. durch Wärmeentzug oder Zufuhr einer anderen Strahlung. Dies dient dazu, den Ballon zu entleeren und das PEG zu entfernen.

Gemäß einer weiteren Ausgestaltung ist die Trokarhülse in Längsrichtung in mindestens zwei Hälften zerlegbar, um die Trokarhülse nach dem Setzen der PEG-Sonde aus dem Einstechkanal in Bauchdecke und Magen zu entfernen. Ein einfaches Abziehen der Trokarhülse von der PEG-Sonde ist insbesondere dann nicht möglich, wenn die PEG-Sonde am proximalen Ende eine Verbreiterung aufweist, die beispielsweise als Handhabe beim Setzen der PEG-Sonde oder als Verschluss für den Ernährungskanal oder für den Füllkanal dient.

Gemäß einer bevorzugten Ausgestaltung ist die Trokarhülse und/oder der Trokar aus Kunststoff. Der Kunststoff ist z.B. PEEK (Polyetherketon).

Gemäß einer weiteren Ausgestaltung ist die Trokarhülse und/oder der Trokar aus Metall, insbesondere aus Edelstahl oder Titan.

Gemäß einer weiteren Ausgestaltung ist die PEG-Sonde aus Kunststoff und/oder Silikon und/oder PUR. Kombinationen der genannten Materialien sind ebenfalls möglich. Zum Beispiel besteht der Ballon aus PUR und der Rest der PEG-Sonde aus einem anderen Kunststoff oder aus Silikon.

Gemäß einer weiteren Ausgestaltung weist der Trokar einen in Längsrichtung durchgehenden Führungskanal für einen Führungsdraht auf. Dies ermöglicht das Setzen einer PEG-Sonde nach Legen eines Führungsdrahtes unter Beobachtung z.B. durch Ultraschall ohne Gastroskopie. Nach Legen des Führungsdrahtes wird der Trokar mit der Trokarhülse durch den Führungsschacht geführt eingeschoben und danach die PEG-Sonde gesetzt. Dieser Trokar kann erfindungsgemäß auch mit herkömmlichen Trokarhülsen und/oder PEG-Sonden verwendet werden.

Mit Hilfe des erfindungsgemäßen Sets kann ein künstlicher Mageneingang gemäß folgender Vorgehensweise hergestellt werden:
1. Zunächst wird ein Gastroskop über den Mund durch die Speiseröhre in den Magen eingeführt und über das Gastroskop Luft in den Magen geblasen, um diesen auszudehnen. Durch die Beleuchtung mit dem Gastroskop vom Magen aus durch die Bauchdecke (Diaphanoskopie) und durch Druck mit dem Finger auf die Bauchdecke wird die Punktionsstelle festgelegt und markiert.
2. Der Punktionsbereich wird mit einer Spritze lokal anästhesiert.
3. An der Punktionsstelle wird mit einem Skalpell nun eine kleine Inzision gemacht.
4. Nun wird der Trokar mit der als Splitting-Hülse ausgeführten Trokarhülse an der Punktionsstelle durch die Bauchdecke in den Magen eingeführt. Dieser Einstich wird von innen mit dem Gastroskop beobachtet.
5. Der Trokar wird aus der Splitting-Hülse entfernt. Die Splitting-Hülse besitzt am proximalen Ende eine Dichtung, damit die Luft nicht aus dem Magen entweichen kann.
6. Als nächstes wird die PEG-Sonde auf den zur Einführung dienenden Stab aufgesetzt und durch die Splitting-Hülse in den Magen eingeführt.
7. Anschließend wird der Ballon am distalen Ende der PEG-Sonde über ein Ventil von außen mit Flüssigkeit gefüllt, um den Magen an die Bauchdecke heranziehen zu können, und ein Herausziehen der PEG-Sonde zu vermeiden. Dieser Vorgang wird mit dem im Magen liegenden Gastroskop überwacht.
8. Die Splitting-Hülse wird nun aus der Bauchdecke herausgezogen, dann aufgebrochen und entfernt.
9. Nun wird der Stab aus der PEG-Sonde herausgezogen.
10. Von außen wird eine Halteplatte für die PEG-Sonde auf der Bauchdecke befestigt.
11. Nun hat man an der PEG-Sonde zwei Anschlüsse: Einen für die Ernährung und einen mit einem Ventil, über das man den Ballon gefüllt hat. Um die beiden Anschlüsse nicht zu verwechseln, kommt nun auf den Anschluss mit dem Zugang zum Ventil eine Schutzkappe, die nur mit einem speziellen Instrument entfernt werden kann.

Die PEG-Sonde kann insbesondere so ausgeführt sein wie die Implantate zur perkutanen Nahrungszufuhr gemäß EP 0 667 763 B1. Insbesondere wird auf die in den Ausführungsbeispielen angegebenen Implantate Bezug genommen.

Nachfolgend ist ein Ausführungsbeispiel der Erfindung anhand der anliegenden Zeichnungen erläutert. In den Zeichnungen zeigen:
- Fig. 1 bis 3 a.) bis e.) und Fig. 4a.) bis: d.) vier Ausführungen von Trokaren mit besonders spitzen Trokarspitzen in Seitenansicht (Fig. 1a bis 4a.), Vorderansicht (Fig. 1b bis 4b), einer vergrößerten Teilansicht von vorn (Fig. lc bis 4c), einer Perspektivansicht (Fig. 1d bis 4d) und in einer vergrößerten Teilansicht von der Seite (Fig. le bis 3e);
- Fig. 5 a.) bis c.): eine Trokarhülse mit Ventil am proximalen Ende (Fig. 5a) und das abgenommene Ventil (Fig. 5b) jeweils in Seitenansicht und das abgenommene Ventil in der Draufsicht (Fig. 5c);
- Fig. 6 a.) und b.): eine PEG-Sonde mit Ballon am distalen Ende und Verschlusselementen am proximalen Ende in Seitenansicht (Fig. 6a) und ein Stab zur Stabilisierung der PEG-Sonde in Seitenansicht (Fig. 6b);
- Fig. 7 a.) und b.): eine PEG-Sonde mit Ballon am distalen Ende und Verschlusselementen am proximalen Ende in Seitenansicht (Fig. 7a) und ein Stab zum Strecken der PEG-Sonde in Seitenansicht (Fig. 7b);
- Fig. 8 a.) bis c.): eine alternative Trokarhülse mit Ventil am proximalen Ende (Fig. 8 a) und das abgenommene Ventil (Fig. 8b) jeweils in Seitenansicht und das angenommene Ventil in der Draufsicht (Fig. 8 c);Fig. 9a) und b.) eine alternative PEG-Sonde mit Ballon am distalen Ende und Verschlusselementen am proximalen Ende in Seitenansicht (Fig. 9a) und ein Stab zur Stabilisierung der PEG-Sonde in Seitenansicht (Fig. 9b);

- Fig. 10 a) und b): alternative PEG-Sonden mit Widerlagern am distalen Ende in Form von Ösen, mit einem Stab zum Strecken mit gabelförmigen Vorsprüngen am distalen Ende in einem Längsschnitt (Fig. 10a) und in Form eines ringförmigen Anschlages zum Aufsetzen eines stumpfen distalen Endes des Stabes in einem Längsschnitt (Fig. 10 b);
- Fig. 11: eine Trokarhülse mit einer eingesetzten Einführhülse mit einer darin angeordneten PEG-Sonde mit elastischem Ballon in einem Längsschnitt.

Bei der nachfolgenden Erläuterung verschiedener Ausführungsbeispiele sind im Wesentlichen übereinstimmende Elemente mit denselben Bezugsziffern versehen.

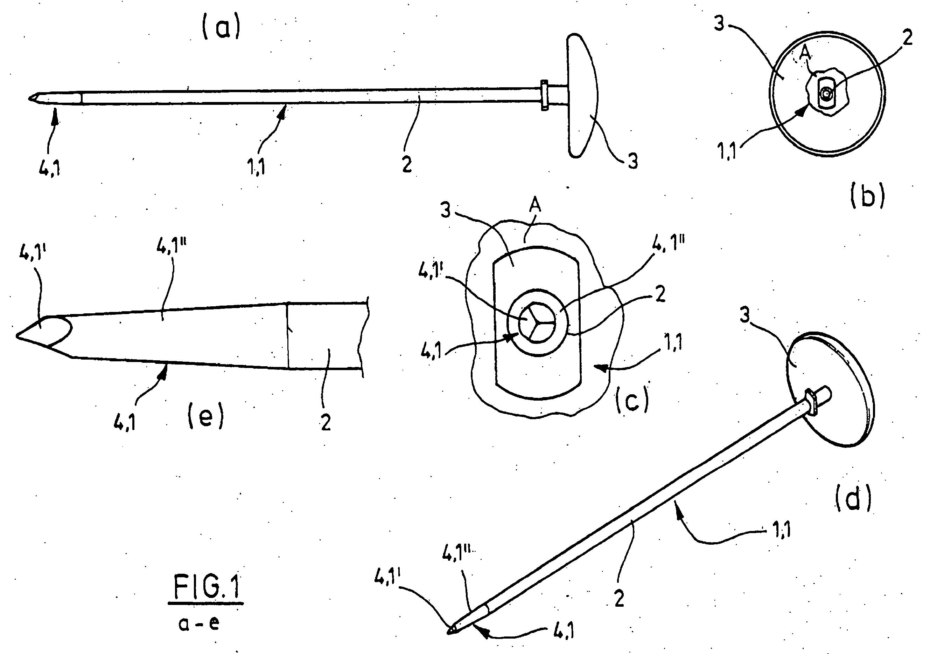

Fig. 1 bis 4 zeigen verschiedene Trokare 1.1, 1.2, 1.3, 1.4, die jeweils einen Stangenabschnitt 2 und eine knaufförmige Handhabe 3 am proximalen Ende des Stangenabschnittes haben.

Gemäß Fig. 1 hat der Trokar 1.1 am distalen Ende des Stangenabschnittes eine Trokarspitze 4.1, die vorne bei 4.1' als Dreikantspitze und hinten bei 4.1" als Konusspitze ausgeführt ist. Der Anschliffwinkel im Bereich 4.1' liegt bei 20° oder darüber. Mit dem Bereich 4.1' wird ein Loch angebohrt. Der halbe Konuswinkel im Bereich 4.1" beträgt 3°. Der halbe Konuswinkel ist maßgeblich für das allmähliche Eindringen und Durchdringen von Bauchdecke und Magenwand.

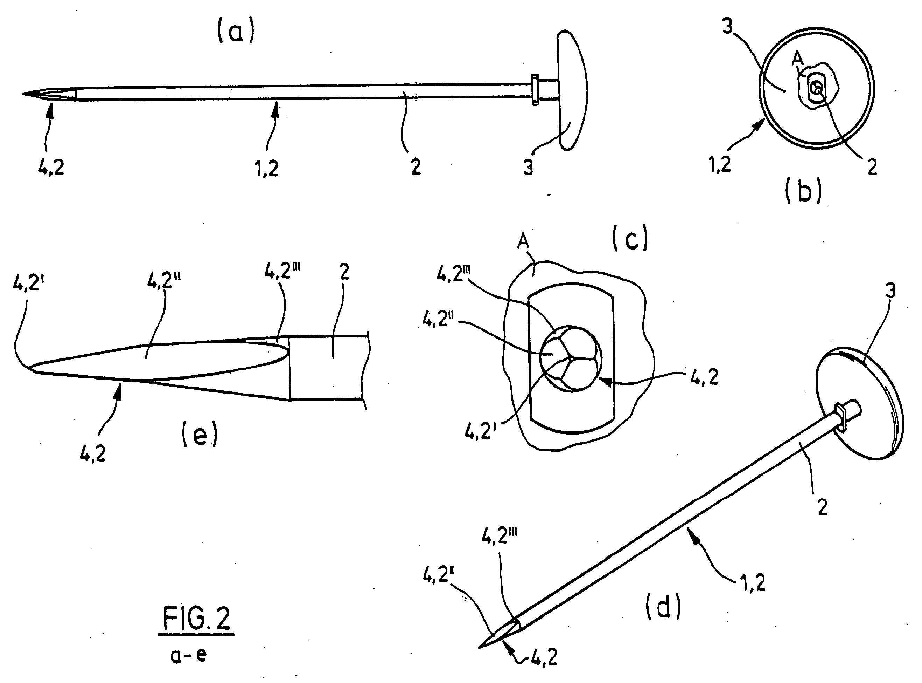

Gemäß Fig. 2 hat der Trokar 1.2 vorne eine Dreikantspitze 4.2. Die Dreikantspitze 4.2 hat ganz vorn eine kurze Anspitzung 4.2' mit einem Konuswinkel von etwa 40°. Der Bereich 4.2' dient dem Anbohren eines Loches. Darauf folgt ein längerer Bereich 4.2", der angeschliffen ist. Der Anschliffwinkel beträgt etwa 6,5°. Dieser Anschliffwinkel ist maßgeblich für das allmähliche Eindringen und Durchdringen von Bauchdecke und Magenwand. Ein Teil des Bereiches 4.2" überlappt sich mit einem konischen Bereich 4.2"'. Der halbe Konuswinkel beträgt 3°. Auch dieser Bereich 4.2"' begünstigt ein allmähliches Eindringen und Durchdringen.

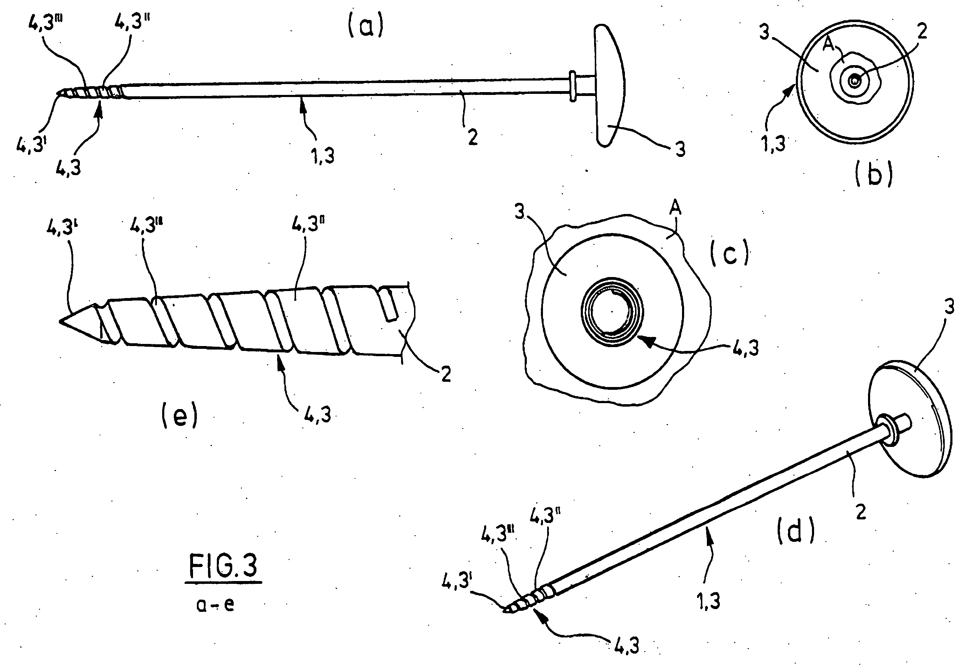

Gemäß Fig. 3 hat der Trokar 1.3 vorne eine Konusspitze 4.3. Die Konusspitze 4.3 ist am vorderen, distalen Ende mit einer konischen Anspitzung 4.3' versehen, deren Konuswinkel etwa 40° beträgt. Dieser Bereich 4.3' dient dem Anbohren eines Loches. Daran schließt sich ein langer, konischer Bereich 4.3" an, der einen halben Konuswinkel von 3° aufweist. Der Bereich 4.3" ist maßgeblich für das allmähliche Eindringen und Durchdringen von Bauchdecke und Magenwand. Der Bereich 4.3" ist mit einem Außengewinde 4.3"' versehen. Dieses unterstützt ein allmähliches Eindringen durch Einschrauben des Trokars 1.3.

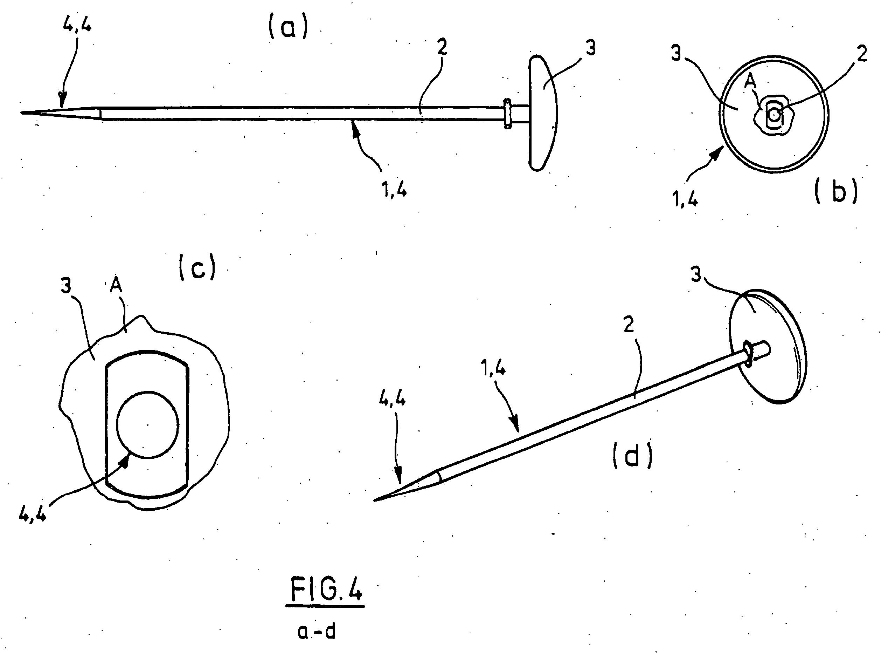

Gemäß Fig. 4 hat der Trokar 1.4 eine Konusspitze 4.4 mit einem halben Konuswinkel von etwa 6,5°. Die Konusspitze 4.4 ist ausschließlich als glatte Konusspitze ausgebildet, d.h. nicht in Bereiche unterteilt. Sie begünstigt ein allmähliches Eindringen und Durchdringen von Bauchdecke und Magenwand.

Die Trokarspitzen 4.1, 4.2, 4.3, 4.4 haben in den für das Eindringen und Durchdringen von Bauchdecke und Magenwand maßgeblichen Bereichen allesamt einen Anschliffwinkel bzw. einen halben Konuswinkel von max. 20°. Unter dem Anschliffwinkel wird der Winkel zwischen den Seitenflächen einer Dreikantspitze zur Mittelachse des Trokars verstanden. Unter dem Konuswinkel wird der Öffnungswinkel des Konus verstanden.

Die Länge der Trokarspitzen 4.1 bis 4.4 beträgt im Beispiel 19 mm. Je nach Durchmesser des Trokars 1.1 bis 1.4 kann sie variieren. Bei einem Trokar mit geringem Durchmesser kann die Länge z.B. nur etwa 5 mm und bei einem Trokar größeren Durchmessers 40 mm betragen. Vorzugsweise ist sie auf 20mm begrenzt. Die gesamte Länge in Axialrichtung der Trokarspitze 4.1 beträgt etwa 19mm. Ferner haben die Trokare 1.1, 1.2, 1.3, 1.4 im Bereich des Stangenabschnittes 2 jeweils einen Durchmesser von 4,35 mm. Durchmesser des Trokars. variiert vorzugsweise im Bereich von 4mm bis 8mm.

Aufgrund der neuartigen, spitzen Ausführung der Trokarspitzen 4.1 bis 4.4 sind die genannten Trokare allmählich in die Bauchdecke und den Magen eines Patienten einführbar.

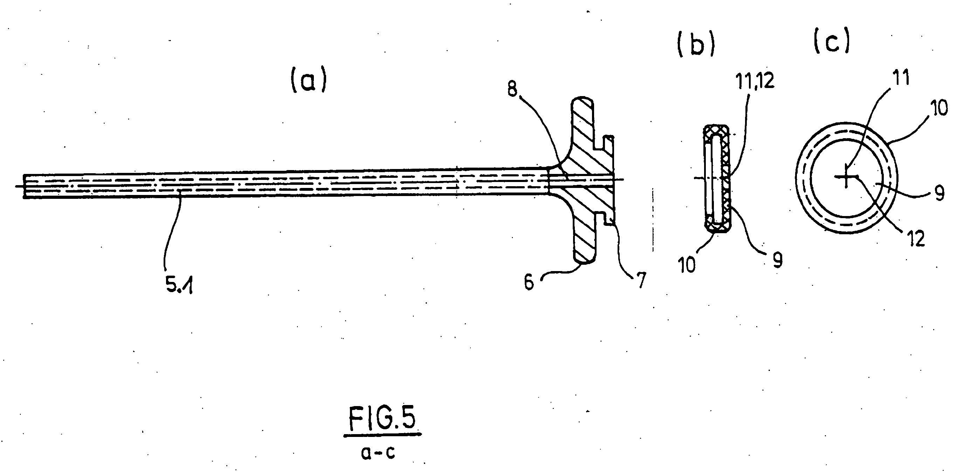

Gemäß Fig. 5 hat eine als Splitting-Kanüle ausgeführte Trokarhülse 5.1 am proximalen Ende einen knebelförmigen Handgriff 6, der an der äußeren Stirnseite einen umlaufenden Flansch 7 aufweist, der im Längsschnitt hakenförmig ist.

Die Trokarhülse 5.1 hat einen Durchgangskanal 8 zum Einführen eines Trokars und einer PEG-Sonde.

Auf dem Flansch 7 ist eine Abdeckscheibe 9 aus elastischem Material platziert, die einen nach außen umgebogenen, umlaufenden Rand 10 aufweist. Mit dem Rand 10 ist die Abdeckscheibe 9 auf den Flansch 7 aufgeschnappt.

Die Abdeckscheibe hat im Zentrum zwei einander kreuzende Schlitze 11, 12, die ein Schlitzventil bilden.

Es können auch drei Schlitze vorhanden sein, die einander in einem Mittelpunkt treffen.

Der Trokar 1 ist durch das Schlitzventil in den Durchgangskanal 8 des Trokars 5 einführbar. Nach Entfernen des Trokars 1 aus der Abdeckscheibe 9 schließen sich die Schlitze und aus dem Durchgangskanal 8 kann ein Überdruck nicht entweichen.

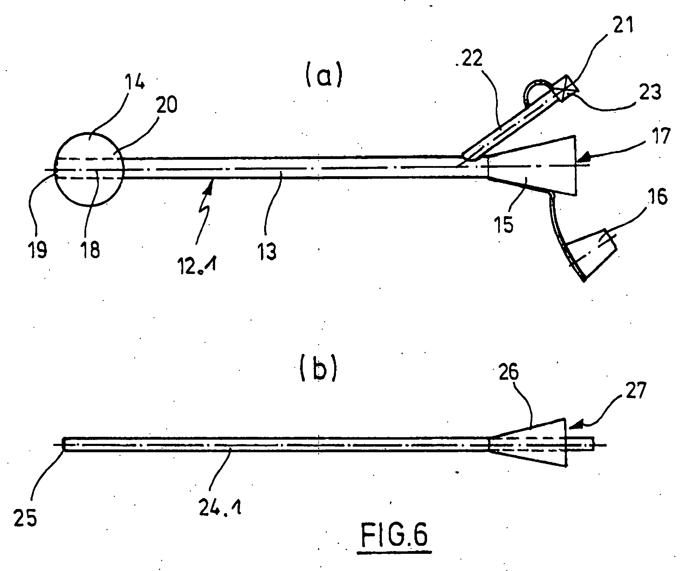

Gemäß Fig. 6a) weist eine PEG-Sonde 12.1 einen rohrförmigen Bereich 13 mit einem Ballon 14 am distalen Ende und einer Verbreiterung 15 am proximalen Ende auf, der ein Verschlussstopfen 16 zugeordnet ist, der in eine Einspeiseöffnung 17 innerhalb der Verbreiterung einsteckbar ist. Der Verschlussstopfen 16 ist über eine Lasche einteilig mit der die Einspeiseöffnung 17 umgebenden Verbreiterung 15 verbunden. Mit der Einspeiseöffnung 17 ist ein Ernährungskanal 18 verbunden, der durch den rohrförmigen Bereich 13 und den Ballon 14 hindurch verläuft und in einer Öffnung 19 am distalen Ende mündet.

Ferner weist die PEG-Sonde 12.1 einen Füllkanal 20 auf, der sich ausgehend von einer Einfüllöffnung 21 in einem seitlich vom proximalen Ende des rohrförmigen Bereiches 13 abstehenden Rohrstück 22 durch den rohrförmigen Bereich 13 hindurch bis zum Kanal 14 erstreckt. Die Einfüllöffnung 21 ist durch ein Ventil 23 verschlossen, das über eine Lasche einteilig mit dem Rohrstück 22 verbunden ist.

Die PEG-Sonde 12.1 besteht aus einem elastischen, flexiblen Material, wie beispielsweise Silikon.

Ferner hat das Set einen Stab 24.1, der ein stumpfes, proximales Ende 25 und eine Handhabe 26 mit einer stirnseitigen Andrückfläche 27 aufweist.

Der Stab 24.1 ist zur Stabilisierung durch die Ernährungsöffnung 17 in den Ernährungskanal 18 einführbar. Danach wird der Stab 24.1 herausgezogen. In dieser Anordnung kann das PEG leicht in eine Trokarhülse 5 eingeführt werden. Nach dem Setzen der PEG-Sonde 12.1 wird der Ballon 14 durch Einfüllen eines Fluids durch die Einfüllöffnung 21 aufgeblasen. Danach wird die Einfüllöffnung 21 durch den Verschluss 23 verschlossen.

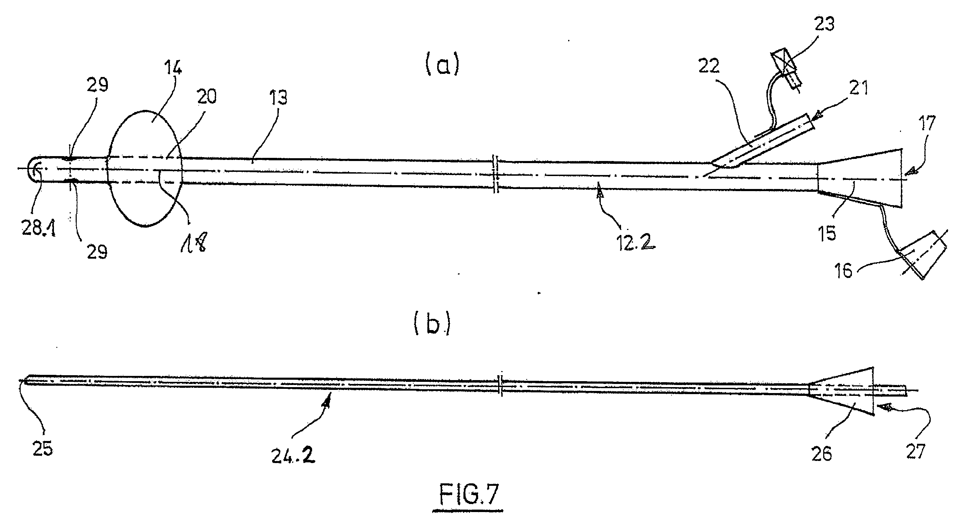

Fig. 7a) zeigt eine PEG-Sonde 12.2, die sich von der zuvor beschriebenen insbesondere durch ihre größere Länge unterscheidet. Ferner sitzt der Ballon 14 nicht direkt am distalen Ende, sondern ist in einem Abstand davon angeordnet. Am distalen Ende ist der rohrförmige Bereich 13 durch einen Boden geschlossen und bildet dort ein Widerlager 28.1. Neben dem Widerlager sind radiale Durchgangsöffnungen 29 vorhanden, die mit dem Ernährungskanal 18 verbunden sind.

Gemäß Fig. 7b) weist ein Streckstab 24.2 im Unterschied zu dem Streckstab von Fig. 6b) insbesondere eine größere Länge auf. Der Streckstab 24.2 ist durch die Einspeiseöffnung 17 in die PEG-Sonde 12.2 einsteckbar, bis er mit seinem stumpfen, distalen Ende 25 gegen das Widerlager 28.1 drückt. Durch Zusammendrücken der Verbreiterung 15 und der Handhabe 26 kann die PEG-Sonde 12.2 gestreckt werden, sodass sie durch eine Trokarhülse 5 hindurchführbar ist, die ein besonders enges Lumen bzw. Querschnitt aufweist.

Zur Vermeidung eines unbeabsichtigten Zusammenfallens des Ballons 14 der PEG-Sonde 12.1 gemäß Fig. 6a) bzw. 12.2 gemäß Fig. 7a) kann der Füllkanal 20 anstatt mit einer Flüssigkeit (z.B. Wasser) mit fließfähigem Medium aufgefüllt werden, das nach dem Auffüllen des Ballons aushärtet. Hierbei kann es sich beispielsweise um einen Klebstoff handeln, insbesondere um einen Klebstoff, der beim oder vor dem Auffüllen des Ballons aus mehreren Komponenten zusammengemischt wird. Das Medium kann nach dem Aushärten starr oder elastisch sein. Bevorzugt ist es elastisch, um das Entnehmen der PEG-Sonde 12.1 bzw. 12.2 zu erleichtern. Hierfür wird der rohrförmige Bereich 13 außerhalb des Körpers des Patienten abgeschnitten und der im Körper verbleibende Teil wird mit dem Ballon 14 mittels einer Fasszange durch die Speiseröhre herausgezogen. Alternativ kann der Ballon 14 am Umfang mit einem elastischen Materialring oder einem anderen elastisch deformierbaren Bereich versehen sein, der nicht mit dem aushärtenden Medium gefüllt wird. Dieser elastisch deformierbare Bereich erleichtert das Herausziehen des distalen Abschnittes der PEG-Sonde 12.1 bzw. 12.2 aus der Speiseröhre.

Bei Anwendung des aushärtenden Mediums kann dieses als Bestandteil eines Sets benutzt werden.

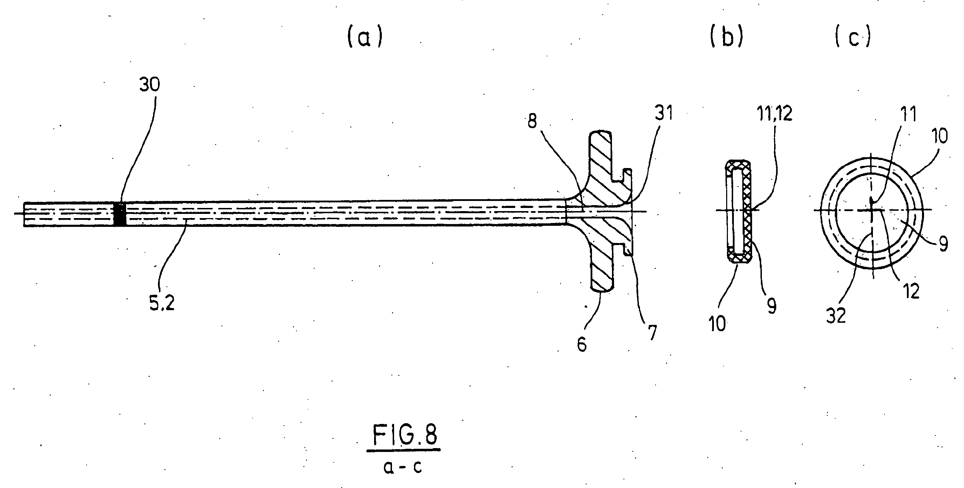

Die Trokarhülse 5.2 von Fig. 8 unterscheidet sich von der Trokarhülse 5.1 dadurch, dass sie in der Nähe des distalen Endes eine Markierung 30 in Form eines auf dem Umfang ringförmig umlaufenden Striches aufweist. Die Markierung 30 kann in das Material des Trokars 5.1 eingebettet oder eine darauf aufgebrachte Lackierung sein. Ferner hat die Trokarhülse 5.2 am proximalen Ende einen konisch sich nach außen hin erweiternden Einführbereich 31 für eine PEG-Sonde. Der Einführbereich 31 erleichtert das Einschieben einer PEG-Sonde.
Eine weitere Besonderheit besteht darin, dass die Abdeckscheibe 9 mit einer Perforationslinie 32 versehen ist, die sich radial über die Abdeckscheibe 9 axial über den Umfang des Randes 10 erstreckt. Entlang der Perforationslinie 32 kann die Abdeckscheibe 9 teilweise aufgetrennt werden, um sie leichter vom Flansch 7 abzunehmen.

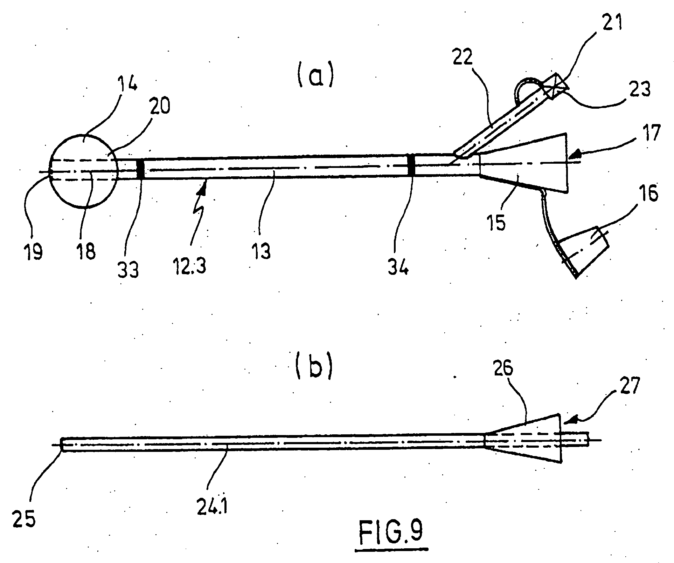

Die PEG-Sonde 12.3 von Fig. 9 unterscheidet sich von der PEG-Sonde 12.1 von Fig. 6 dadurch, dass der rohrförmige Bereich 13 mit einer distalen Markierung 33 und einer proximalen Markierung 34 versehen sind. Diese sind jeweils über den Umfang des rohrförmigen Bereiches 12 verlaufende, ringförmige Linien ausgebildet. Diese können in das Material der PEG-Sonde 12.1 eingebettet oder auflackiert sein.

Die distale Markierung 33 ist in einem kurzen Abstand von etwa 1cm vom Ballon 14 angeordnet. Die proximale Markierung 34 ist in verhältnismäßig kurzem Abstand von etwa einem oder mehreren Zentimetern von der Verbreiterung 15 angeordnet. Der Operateur kann mittels des Gastrokopes gut erkennen, wenn die Markierung 33 aus einer Trokarhülse austritt. Dann ist die PEG-Sonde 12.3 hinreichend in die Trokarhülse eingeschoben. Man kann das hinreichende Einschieben der PEG-Sonde 12.3 in die Trokarhülse aber auch daran erkennen, dass die Markierung 34 das proximale Ende der Trokarhülse erreicht. Dies reicht zur Orientierung aus, wenn die Trokarhülse korrekt in Bauchdecke und Magen eingesetzt ist, was mittels des Gastroskopes kontrolliert werden kann.

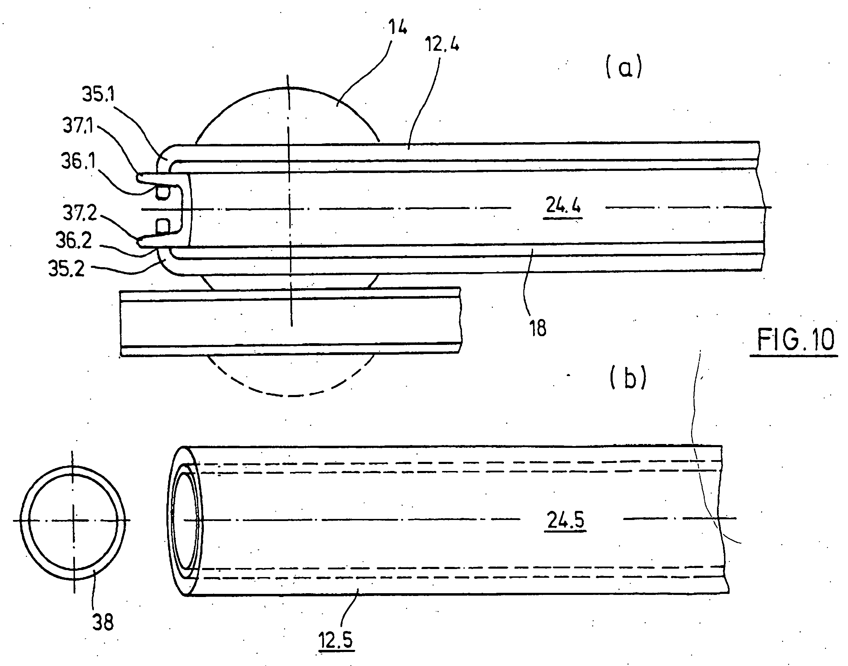

Gemäß Fig. 10a) hat eine PEG-Sonde 12.4 am distalen Ende zwei flexible Laschen 35.1, 35.2, die jeweils ein Loch 36.1, 36.2 aufweisen und jeweils eine Öse bilden. Ein Stab 24.4 ist am distalen Ende mit zwei gabelförmigen Vorsprüngen 37.1, 37.2 versehen. Die flexiblen Laschen 35.1, 355.2 sind in die distale Öffnung des Ernährungskanals 18 faltbar, sodass der Stab 24.4 mit den Vorsprüngen 37.1, 37.2 in die Löcher 36.1, 36.2 einsetzbar ist. Auf diese Weise wird der Streckstab 24.3 distal gehalten und kann zum Dehnen der elastischen PEG-Sonde 12.4 genutzt werden.

Die Ausführung von Fig. 10b) unterscheidet sich von der vorbeschriebenen dadurch, dass die PEG-Sonde 12.5 am distalen Ende einen eingesetzten Ring 38 aufweist, der in der Zeichnung zur Veranschaulichung aus der PEG-Sonde 12.5 herausgenommen ist. Der Ring 38 ist in der PEG-Sonde 12.4 so fixiert, dass der Stab 24.5 mit einem stumpfen Ende gegen den Ring 39 drückbar ist, der ein Widerlager bildet. Somit ist auch die elastische PEG-Sonde 12.5 mittels des Streckstabes 24.5 streckbar.

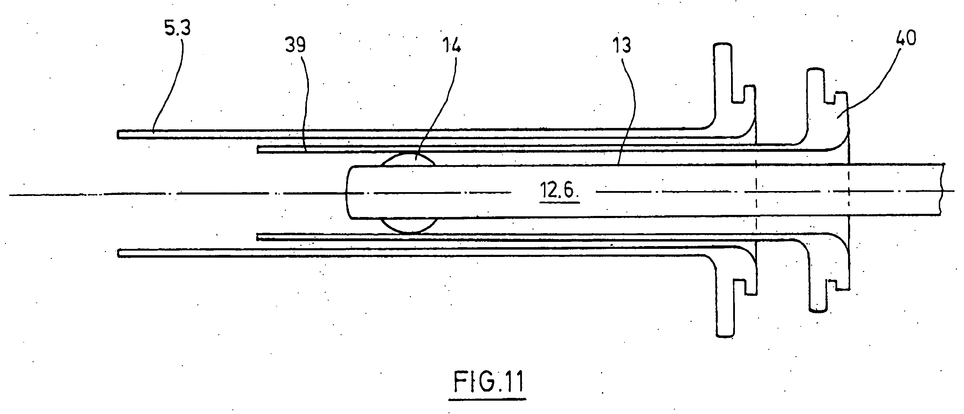

Bei dem Set von Fig. 11 hat die PEG-Sonde 12.6 einen eng an den rohrförmigen Bereich anliegenden Ballon 14. Dieser ist beispielsweise ein Ballon 14, der insgesamt aus einem elastischen Material besteht oder mit einem elastischen Material gefüllte Hülle aufweist. Alternativ handelt es sich um einen Ballon 14, der aus einem flexiblen Material geringer Dehnbarkeit besteht. In letzerem Falle kann der Ballon 14 mittels eines Gels, das sich im Magen eines Patienten auflöst, in der gezeigten Lage an dem rohrförmigen Bereich 13 fixiert sein.

Der Ballon 14 ist von einer Einführhülse 39 in dem eng an dem rohrförmigen Bereich 13 anliegenden Zustand fixiert. Die Einführhülse 39 hat am proximalen Ende eine weitere Handhabe 40 in Form eines sich radial nach außen verbreiternden Bereiches.

In der Einführhülse 39 ist die PEG-Sonde 12.6 in eine Trokarhülse 5.3 eingeschoben. Hierbei wird der Ballon 14 von der Einführhülse 39 in der komprimierten Lage fixiert. Nach Abziehen der Einführhülse 39 kann diese in Längsrichtung geteilt und abgenommen werden, auch wenn die PEG-Sonde 12.6 am proximalen Ende mit einer Verbreiterung versehen ist. Falls der Ballon 14 aus einem elastischen Material besteht oder dieses enthält, weitet er sich radial auf, sodass er am Innenumfang der Trokarhülse 5.3 anliegt. Bei einer Ausführung des Ballons 14 aus flexiblem, bevorzugt nicht dehnbarem Material liegt der Ballon 14 weiterhin an der PEG-Sonde 12.6 an.

Anschließend kann die PEG-Sonde 12.6 so weit in die Trokarhülse 5.3 eingeschoben werden, dass der Ballon aus dem distalen Ende derselben austritt. Ein elastischer Ballon expandiert dann selbsttätig, sodass er sich an der Magenwand abstützen kann. Ein Ballon 14 aus einem flexiblen Material wird durch einen Füllkanal der PEG-Sonde 12.6 mit einem Fluid gefüllt, sodass er aufgebläht wird und sich an der Magenwand abstützen kann.

## Patentansprüche

1. Set zur Schaffung eines künstlichen Mageneinganges, umfassend einen Trokar (1) mit Trokarhülse (5) zum Einstechen durch die Bauchdecke in den Magen und eine nach Entnahme des Trokars (1) aus der Trokarhülse (5) in diese einführbare PEG-Sonde (12), wobei der Trokar eine Trokarspitze (4) aufweist, die eine Konusspitze (4") ist, bei der der halbe Konuswinkel 5° bis 12° beträgt und der Trokar (1) einen Durchmesser von 4 bis 8 mm aufweist.

2. Set nach Anspruch 1, bei dem die Trokarspitze (4) eine Länge von 8 mm bis 22 mm aufweist und/oder bei dem die Trokarspitze (4) mit einem Außengewinde versehen ist.

3. Set nach Anspruch 1 oder 2, umfassend eine PEG-Sonde (12) mit einer in den Magen einsetzbaren, distalen Erweiterung in Form eines Ballons (14) aus einem flexiblen Material, das nicht dehnbar ist oder allenfalls eine geringe Dehnbarkeit aufweist, die geringer als die Dehnbarkeit von Silikon ist, die distal mindestens eine Öffnung (19) aufweist und einen proximal daran anschließenden rohrförmigen Bereich (13), wobei ein Emährungskanal (18) von einer proximalen Ernährungsöffnung des rohrförmigen Bereichs aus durch den rohrförmigen Bereich bis zu der mindestens einen Öffnung (19) erstreckt ist und der Ballon mit einem Füllkanal verbunden ist, der sich entlang des rohrförmigen Bereichs bis zum proximalen Ende erstreckt und eine Einfüllöffnung mit einem Verschluss (23) aufweist, wobei der Ballon (14) im kollabierten Zustand am rohrförmigen Bereich anliegt und in diesem Zustand durch Sicherungsmittel auf dem rohrförmigen Bereich (13) gesichert ist.

4. Set nach Anspruch 3, bei dem der Ballon aus PUR hergestellt ist.

5. Set nach einem der Ansprüche 1 bis 4, wobei die Trokarhülse (5) proximal ein Ventil (9) aufweist, durch das der Trokar (1) in die Trokarhülse (5) einführbar ist und das nach Entnahme des Trokars (1) aus der Trokarhülse (5) gasdicht abschließt, wobei das Ventil (9) mindestens eine Schwächungslinie (32) zum zumindest teilweisen Auftrennen des Ventils (9) aufweist, deren Auftrennen das Entfernen des Ventils (9) von der Trokarhülse (5) erleichtert.

6. Set nach Anspruch 5, bei dem die Trokarhülse (5) und das Ventil (9) Haltemittel zum lösbaren Halten des Ventils (9) an der Trokarhülse (5) aufweisen und/oder bei dem das Ventil (9) und die Trokarhülse (5) Schnappmittel zum Aufschnappen des Ventils (9) auf die Trokarhülse (5) haben und/oder bei dem die Abdeckscheibe (9) einen nach außen umgebogenen, umlaufenden Rand (10) und die Trokarhülse (5) einen umlaufenden Flansch (7) am proximalen Ende zum Aufschnappen der Abdeckscheibe (9) mit dem umlaufenden Rand (10) aufweist und/oder bei dem das Ventil eine Abdeckscheibe (9) aus elastischem Material oder aus einem Material mit einem Memoryeffekt umfasst, die randseitig an der Trokarhülse (5) festgelegt ist und den Durchgangskanal (8) der Trokarhülse (5) versperrt und durch die der Trokar (1) zum Einführen in den Durchgangskanal (8) einstechbar ist, wobei die Einstechstelle (11, 12) in der Abdeckscheibe (9) nach Herausziehen des Trokars aufgrund elastischer Rückstellkräfte oder des Memoryeffektes schließt.

7. Set nach Anspruch 5 oder 6, bei dem das Ventil (9) mindestens einen Schlitz (10, 11) zum Einstechen des Trokars (1) aufweist und/oder bei dem die Anzahl der Schlitze (10, 11) in der Abdeckscheibe (9) der Anzahl der Kanten der Mehrkantspitze (4) des Trokars (1) entspricht, wobei die Schlitze von einer zentralen Schnittstelle ausgehend entsprechend der Ausrichtung der Kanten der Mehrkantspitze des Trokars (1) ausgerichtet sind.

8. Set nach einem der Ansprüche 5 bis 7, bei dem das Ventil randseitig eine Aufreißlasche aufweist und/oder bei dem die Schwächungslinie in Radialrichtung und/oder Umfangsrichtung der Abdeckscheibe verläuft.

9. Set nach einem der Ansprüche 1 bis 8, wobei die Trokarhülse (5) in einem Abstand vom distalen Ende am Umfang eine Markierung aufweist, zum Anzeigen, ob die Trokarhülse (5) tief genug in Bauchdecke und Magen eingeschoben ist.

10. Set nach Anspruch 9, bei dem die Markierung in einem Abstand von 2 bis 5 cm vom distalen Ende der Trokarhülse angeordnet ist und/oder bei dem die Markierung ein auf dem Umfang der Trokarhülse angeordneter Strich ist.

11. Set nach einem der Ansprüche 1 bis 10, umfassend eine PEG-Sonde (12) aus flexiblem Material, die eine in den Magen einsetzbare, distale Erweiterung (14) hat, die mindestens eine distale Öffnung (19) aufweist, und einen proximal an die Erweiterung anschließenden rohrförmigen Bereich (13), wobei von einer proximalen Ernährungsöffnung (17) des rohrförmigen Bereichs (13) aus durch den rohrförmigen Bereich bis zu der mindestens einen Öffnung (19) ein Ernährungskanal (18) verläuft, und einen Stab (24) mit einem distalen Ende (25) und einer Handhabe (26) am proximalen Ende, der mit dem distalen Ende (25) in den Ernährungskanal (18) einschiebbar ist.

12. Set nach Anspruch 11, bei dem die PEG-Sonde (12) aus einem elastischen Material besteht und/oder bei dem die Länge des Stabes die Länge der PEG-Sonde übersteigt, so dass die PEG-Sonde mittels des eingesetzten Stabes streckbar ist und/oder bei dem die PEG-Sonde (12) am distalen Ende ein Widerlager (28) für den Stab (24) aufweist und/oder bei dem das Widerlager ein Boden der PEG-Sonde (12) oder ein Absatz im Ernährungskanal (18) ist, auf den der Stab mit dem distalen Ende aufsetzbar ist, oder mindestens eine Öse am distalen Ende der PEG-Sonde (12) ist, in die der Stab mit einem Vorsprung am distalen Ende einsetzbar ist, oder ein sich im Milieu des Magens auflösender Stopfen im Ernährungskanal (18) ist.

13. Set nach Anspruch 11 oder 12, bei dem die PEG-Sonde (12) am proximalen Ende eine Verbreiterung (26) aufweist und/oder bei dem die PEG-Sonde (12) am proximalen Ende einen Verschluss (16) aufweist.

14. Set nach einem der Ansprüche 11 bis 13, bei dem die PEG-Sonde (12) am distalen Ende (25) einen Ballon (14) und einen damit verbundenen und entlang des rohrförmigen Bereichs (13) bis zum proximalen Ende erstreckten Füllkanal (20) mit einem Verschluss (23) an einer proximalen Einfüllöffnung (21) aufweist, wobei durch den Füllkanal (10) ein Fluid zum Aufpumpen des Ballons (14) einfüllbar ist.

15. Set nach einem der Ansprüche 1 bis 14, umfassend eine PEG-Sonde (12) mit einer in den Magen einsetzbaren, distalen Erweiterung in Form eines Ballons (14), die mindestens eine distale Öffnung (19) aufweist und einem proximal daran anschließenden rohrförmigen Bereich (13), wobei ein Ernährungskanal (18) von einer proximalen Ernährungsöffnung (17) des rohrförmigen Bereichs (13) aus durch den rohrförmigen Bereich bis zu der mindestens einen Öffnung (19) erstreckt ist und der Ballon (14) mit einem Füllkanal (20) verbunden ist, der sich entlang des rohrförmigen Bereiches (13) bis zum proximalen Ende erstreckt und eine Einfüllöffnung (21) mit einem Verschluss (23) aufweist, wobei der Verschluss (23) ein den Querschnitt der Einfüllöffnung (21) versperrendes, entfernbares Schließelement aufweist, das Schließelement sowie die PEG-Sonde am proximalen Ende Mittel zum Verriegeln des Schließelementes an der PEG-Sonde aufweisen und das Schließelement Mittel zum Ansetzen eines Schlüssels zum Zwecke der Aufhebung der Verriegelung des Schließelementes mit der PEG-Sonde (12) aufweist.

16. Set nach Anspruch 15, bei dem das Schließelement ein Gewindestift ist, der in ein Innengewinde in der Einfüllöffnung (21) einschraubbar ist und/oder bei dem die Mittel zum Ansetzen eines Schlüssels ein Werkzeugangriff und der Schlüssel ein an den Werkzeugangriff ansetzbares Werkzeug sind.

17. Set nach einem der Ansprüche 15 bis 16, bei dem der Ballon (14) und/oder die PEG-Sonde (12) aus flexiblem oder elastisch verformbarem Material besteht.

18. Set nach einem der Ansprüche 1 bis 17, umfassend eine PEG-Sonde (12) mit einer in den Magen einsetzbaren, distalen Erweiterung (14), die distal mindestens eine Öffnung (19) aufweist und einen proximal daran anschließenden rohrförmigen Bereich (13), wobei ein Ernährungskanal (18) von einer proximalen Ernährungsöffnung des rohrförmigen Bereichs aus durch den rohrförmigen Bereich bis zu der mindestens einen Öffnung (19) erstreckt, wobei die PEG-Sonde (12) in einem Abstand von der distalen Erweiterung und/oder vom proximalen Ende eine Markierung (33, 34) aufweist, zum Anzeigen, ob die PEG-Sonde (12) tief genug in die Trokarhülse eingeschoben ist.

19. Set nach Anspruch 18, bei dem die PEG-Sonde (12) eine distale Erweiterung in Form eines Ballons (14) ausweist.

20. Set nach einem der Ansprüche 1 bis 19, umfassend eine PEG-Sonde (12) mit einer in den Magen einsetzbaren, distalen Erweiterung in Form eines elastischen Ballons (14) mit einer den Ballon umgebenden Einführhülse (39), in der der Ballon (14) radial elastisch zusammengepresst angeordnet ist, wobei die Einführhülse (39) mit der darin angeordneten PEG-Sonde (12) in die Trokarhülse (5) einführbar ist und die PEG-Sonde (12) mit dem Ballon (14) in distaler Richtung aus der Einführhülse (39) heraus und aus der Trokarhülse herausschiebbar ist, so dass der Ballon (14) expandiert.

21. Set nach Anspruch 20, bei dem der Ballon (14) mit einem elastischen Schaumstoff gefüllt ist und/oder bei dem die Einführhülse (39) eine in Längsrichtung teilbare Hülse ist, um die Einführhülse (39) nach dem Herausschieben des Ballons (14) aus der Trokarhülse (5) zu entfernen.

22. Set gemäß einem der Ansprüche 1 bis 21, wobei der Trokar einen in seiner Längsrichtung erstreckten Führungskanal für einen Führungsdraht aufweist.

## Claims

1. A kit providing an artificial stomach entrance, comprising a trocar (1) with trocar sleeve (5) for penetration through the abdominal wall into the stomach and a PEG tube (12) insertable into it after removal of the trocar (1) from the trocar sleeve (5), wherein the trocar has a trocar tip (4), which is a conical tip (4"), in which the half conical angle is 5° to 12° and the trocar (1) has a diameter of 4 to 8 mm.

2. The kit according to claim 2, in which the trocar tip (4) has a length of 8 mm to 22 mm and/or in which the trocar tip (4) is provided with an external thread.

3. The kit for providing an artificial stomach entrance, comprising a PEG tube (12) with a distal extension insertable into the stomach in the form of a balloon (14) made of a flexible material that is not stretchable or if need has only slight stretchability, which is less stretchable than silicon, which has at least one opening (19) distally and a proximally connecting tubular area (13), wherein a feeding channel (18) extends from a proximal feeding opening of the tubular area through the tubular area up to the at least one opening (19) and the balloon is connected with a fill channel, which extends along the tubular area up to the proximal end and has a fill opening with a seal (23), and wherein the balloon in a collapsed state engages with the tubular area and in this state is secured by safety means on the tubular area (13).

4. The kit according to claim 1, in which the balloon is made of PUR.

5. The kit according to one of the claims 1 to 4, wherein the trocar sleeve (5) has a valve (9) proximally, through which the trocar (1) can be inserted into the trocar sleeve (5) and which seals airtight after removal of the trocar (1) from the trocar sleeve (5), wherein the valve (9) has at least one weakening line (32) for at least partial ripping of the valve (9), the ripping of which facilitates the removal of the valve (9) from the trocar sleeve (5).

6. The kit according to claim 5, in which the trocar sleeve (5) and the valve (9) have holding means for releasably holding the valve (9) on the trocar sleeve (5) and/or in which the valve (9) and the trocar sleeve (5) have snapping means for snapping the valve (9) onto the trocar sleeve (5) and/or in which the cover plate (9) has an outwardly bent, circumferential edge (10) and the trocar sleeve (5) a circumferential flange (7) on the proximal end for snapping the cover plate (9) to the circumferential edge (10) and/or in which the valve comprises a cover plate (9) made of an elastic material or a material with a memory effect, which is affixed to the trocar sleeve (5) on the edge and blocks the passage channel (8) of the trocar sleeve (5) and through which the trocar (1) is piercable for insertion into the passage channel (8), wherein the penetration point (11, 12) in the cover plate (9) closes after removal of the trocar due to the elastic reset forces or the memory effect.

7. The kit according to claim 5 or 6, in which the valve (9) has at least one slit (10, 11) for inserting the trocar (1) and/or in which the number of slits (10, 11) in the cover plate (9) matches the number of edges of the multi-edge tip (4) of the trocar (1), wherein the slits are aligned starting from a central cutting point according to the alignment of the edges of the multi-edge tip of the trocar (1).

8. The kit according to one of claims 5 through 7, in which the valve has a pull tab on the edge side and/or in which the weakening line runs in the radial direction and/or circumferential direction of the cover plate.

9. The kit according to one of the claims 1 to 8, wherein the trocar sleeve (5) has a marking on the perimeter at a distance from the distal end for displaying whether the trocar sleeve (5) is pushed deep enough into the abdominal wall and stomach.

10. The kit according to claim 9, in which the marking is arranged at a distance of 2 to 5 cm from the distal end of the trocar sleeve and/or in which the marking is a line arranged on the perimeter of the trocar sleeve.

11. The kit according to one of the claims 1 to 10, comprising a PEG tube (12) insertable into it after removal of the trocar from the trocar sleeve made of a flexible material, which has a distal extension (14) insertable into the stomach, which has at least one distal opening (19), and a tubular area (13) connecting proximally to the extension, wherein a feeding channel (18) progresses from a proximal feeding opening (17) of the tubular area (13) through the tubular area up to the at least one opening (19), and a rod (24) with a distal end (25) and a handle (26) on the proximal end, which can be pushed into the feeding channel (18) with the distal end (25).

12. The kit according to claim 11, in which the PEG tube (12) is made of an elastic material and/or in which the length of the rod exceeds the length of the PEG tube so that the PEG tube is stretchable by means of the inserted rod and/or in which the PEG tube (12) has an abutment (28) for the rod (24) on the distal end and/or in which the abutment is a bottom of the PEG tube (12) or a step in the feeding channel (18), onto which the rod with the distal end can be placed, or at least one eyelet on the distal end of the PEG tube (12), into which the rod with a projection on the distal end can be inserted, or a plug in the feeding channel (18) dissolving in the environment of the stomach.

13. The kit according to claim 11 or 12, in which the PEG tube (12) has a broadening (26) on the proximal end and/or in which the PEG tube (12) has a seal (16) on the proximal end.

14. The kit according to one of claims 11 through 13, in which the PEG tube (12) on the distal end (25) has a balloon (14) and a fill channel (20) connected with it and extended along the tubular area (13) up to the proximal end with a seal (23) on a proximal fill opening (21), wherein a fluid for pumping up the balloon (14) can be filled through the fill channel (10).

15. The kit according to one of the claims 1 to 14, comprising a PEG tube (12) with a distal extension insertable into the stomach in the form of a balloon (14), which has at least one opening (19) and a proximally connecting tubular area (13), wherein a feeding channel (18) extends from a proximal feeding opening (17) of the tubular area (13) through the tubular area up to the at least one opening (19) and the balloon (14) is connected with a fill channel (20), which extends along the tubular area (13) up to the proximal end and has a fill opening (21) with a seal (23), wherein the seal (23) has a removable closing element blocking the cross-section of the fill opening (21), the closing element and the PEG tube on the proximal end have means for locking the closing element on the PEG tube and the closing element has a means for the use of a key for the purpose of cancelling the lock of the closing element with the PEG tube (12).

16. The kit according to claim 15, in which the closing element is a threaded pin, which is screwable into an internal thread in the fill opening (21) and/or in which the means for applying a key is a tool application point and the key is a tool that can be placed on the tool application point.

17. The kit according to one of claims 15 through 16, in which the balloon (14) and/or the PEG tube (12) are made of a flexible or elastically deformable material.

18. The kit according to one of the claims 1 to 17, comprising a PEG tube (12) insertable into it after removal of the trocar from the trocar sleeve with a distal extension (14) insertable into the stomach, which has distally at least one opening (19), and a proximally connecting tubular area (13), wherein a feeding channel (18) extends from a proximal feeding opening of the tubular area through the tubular area up to the at least one opening (19), wherein the PEG tube (12) has a marking (33, 34) at a distance from the distal extension and/or the proximal end, for displaying whether the PEG tube (12) is pushed deep enough into the trocar sleeve.

19. The kit according to claim 18, in which the PEG tube (12) has a distal extension in the form of a balloon (14).

20. The kit according to one of the claims 1to 19, comprising a PEG tube (12) insertable into it after removal of the trocar from the trocar sleeve with a distal extension insertable into the stomach in the form of an elastic balloon (14) with an insertion sleeve (39) surrounding the balloon, in which the balloon (14) is arranged pressed together radially elastically, wherein the insertion sleeve (39) with the PEG tube (12) arranged in it is insertable into the trocar sleeve (5) and the PEG tube (12) with the balloon (14) can be pushed out of the insertion sleeve (39) and out of the trocar sleeve in the distal direction so that the balloon (14) expands.

21. The kit according to claim 20, in which the balloon (14) is filled with an elastic foam and/or in which the insertion sleeve (39) is a sleeve dividable in the longitudinal direction in order to remove the insertion sleeve (39) from the trocar sleeve (5) after the pushing out of the balloon (14).

22. The kit according to one of the claims 1 to 21, wherein the trocar has a guide channel for a guide wire extended in its longitudinal direction.

## Revendications

1. Kit pour la création d'une gastrostomie, comprenant un trocart (1) avec une douille de trocart (5) permettant de percer la paroi abdominale jusqu'à l'estomac, ainsi qu'une sonde PEG (12) insérable dans la douille de trocart (5) après le retrait du trocart (1) hors de celle-ci, le trocart présentant une pointe de trocart (4) consistant en une pointe conique (4"), dans laquelle le demi-angle de cône mesure de 5° à 12° et le trocart (1) présente un diamètre de 4 à 8 mm.

2. Kit selon la revendication 1, dans lequel la pointe de trocart (4) présente une longueur de 8 mm à 22 mm et/ou dans lequel la pointe de trocart (4) est pourvue d'un filetage extérieur.

3. Kit selon la revendication 1 ou 2, comprenant une sonde PEG (12) avec une extension distale insérable dans l'estomac, sous la forme d'un ballon (14) constitué d'un matériau souple non extensible ou présentant du moins une faible extensibilité, notamment inférieure à l'extensibilité du silicone, laquelle présente au moins une ouverture (19) distale suivie d'une région tubulaire (13) proximale, un canal d'alimentation (18) s'étendant à partir de l'ouverture d'alimentation proximale de la région tubulaire, à travers la région tubulaire et jusqu'à l'au moins une ouverture (19), et le ballon étant relié à un canal de remplissage s'étendant le long de la région tubulaire jusqu'à l'extrémité proximale et présentant une ouverture de remplissage avec un moyen de fermeture (23), le ballon (14) s'appliquant contre la région tubulaire dans l'état plié et étant fixé dans cet état par des moyens de fixation sur la région tubulaire (13).

4. Kit selon la revendication 3, dans lequel le ballon est constitué de PUR.

5. Kit selon l'une des revendications 1 à 4, dans lequel la douille de trocart (5) présente une soupape (9) à l'extrémité proximale, à travers laquelle le trocart (1) peut être inséré dans la douille de trocart (5) et laquelle se ferme de façon étanche au gaz après le retrait du trocart (1) hors de la douille de trocart (5), la soupape (9) présentant au moins une ligne d'affaiblissement (32) pour séparer au moins partiellement la soupape (9), dont l'ouverture facilite le retrait de la soupape (9) par rapport à la douille de trocart (5).

6. Kit selon la revendication 5, dans lequel la douille de trocart (5) et la soupape (9) présentent des moyens de maintien permettant de maintenir la soupape (9) de façon détachable contre la douille de trocart (5), et/ou dans lequel la soupape (9) et la douille de trocart (5) ont des moyens d'encliquetage destinés à encliqueter la soupape (9) sur la douille de trocart (5), et/ou dans lequel le disque de recouvrement (9) présente un bord périphérique (10) recourbé vers l'extérieur et la douille de trocart (5) présente une bride périphérique (7) à l'extrémité proximale pour l'encliquetage du disque de recouvrement (9) avec le bord périphérique (10), et/ou dans lequel la soupape comporte un disque de recouvrement (9) constitué d'un matériau élastique ou d'un matériau à effet de mémoire, lequel est fixé sur le bord de la douille de trocart (5) et bloque le canal de passage (8) de la douille de trocart (5), et à travers lequel le trocart (1) peut percer pour s'insérer dans le canal de passage (8), le point d'insertion (11, 12) dans le disque de recouvrement (9) se refermant après le retrait du trocart du fait de forces de rappel élastiques ou de l'effet de mémoire.

7. Kit selon la revendication 5 ou 6, dans lequel la soupape (9) présente au moins une fente (10, 11) pour l'insertion du trocart (1), et/ou dans lequel le nombre de fentes (10, 11) dans le disque de recouvrement (9) correspond au nombre de chants de la pointe polygonale (4) du trocart (1), les fentes étant orientées conformément à l'orientation des chants de la pointe polygonale du trocart (1) en partant d'une interface centrale.

8. Kit selon l'une des revendications 5 à 7, dans lequel la soupape présente une languette déchirable sur son bord, et/ou dans lequel la ligne d'affaiblissement s'étend dans la direction radiale et/ou dans la direction circonférentielle du disque de recouvrement.

9. Kit selon l'une des revendications 1 à 8, dans lequel la douille de trocart (5) présente un marquage sur son pourtour, à une distance de l'extrémité distale, pour indiquer si la douille de trocart (5) est enfoncée suffisamment profondément dans la paroi abdominale et l'estomac.

10. Kit selon la revendication 9, dans lequel le marquage se trouve à une distance de 2 à 5 cm de l'extrémité distale de la douille de trocart, et/ou dans lequel le marquage est un trait disposé sur le pourtour de la douille de trocart.

11. Kit selon l'une des revendications 1 à 10, comprenant une sonde PEG (12) constituée d'un matériau souple, laquelle présente une extension distale (14) susceptible d'être insérée dans l'estomac et présentant au moins une ouverture distale (19), ainsi qu'une région tubulaire (13) disposée à la suite de l'extension de façon proximale, dans lequel un canal d'alimentation (18) s'étend à partir d'une ouverture d'alimentation proximale (17) de la région tubulaire (13), à travers la région tubulaire et jusqu'à l'au moins une ouverture (19), et une tige (24) avec une extrémité distale (25) et une zone de prise (26) à l'extrémité proximale, laquelle peut être insérée avec son extrémité distale (25) dans le canal d'alimentation (18).

12. Kit selon la revendication 11, dans lequel la sonde PEG (12) est constituée d'un matériau élastique et/ou dans lequel la longueur de la tige dépasse la longueur de la sonde PEG, de sorte que la sonde PEG est extensible grâce à la tige insérée, et/ou dans lequel la sonde PEG (12) présente une butée (28) pour la tige (24) à l'extrémité distale, et/ou dans lequel la butée est un fond de la sonde PEG (12) ou un épaulement dans le canal d'alimentation (18), sur lequel la tige peut être placée avec son extrémité distale, ou est du moins un oeillet à l'extrémité distale de la sonde PEG (12), dans lequel la tige peut être insérée avec une saillie à l'extrémité distale, ou est un bouchon dans le canal d'alimentation (18) soluble dans l'estomac.

13. Kit selon la revendication 11 ou 12, dans lequel la sonde PEG (12) présente un élargissement (26) à son extrémité proximale, et/ou dans lequel la sonde PEG (12) présente un moyen de fermeture (16) à son extrémité proximale.

14. Kit selon l'une des revendications 11 à 13, dans lequel la sonde PEG (12) présente un ballon (14) à l'extrémité distale (25) et un canal de remplissage (20) relié à celui-ci et s'étendant le long de la région tubulaire (13), jusqu'à l'extrémité proximale, avec un moyen de fermeture (23) au niveau d'une ouverture de remplissage proximale (21), un fluide pouvant être alimenté à travers le canal de remplissage (10) pour le gonflage du ballon (14).

15. Kit selon l'une des revendications 1 à 14, comprenant une sonde PEG (12) avec une extension distale insérable dans l'estomac sous la forme d'un ballon (14), laquelle présente au moins une ouverture distale (19) suivie d'une région tubulaire proximale (13), un canal d'alimentation (18) s'étendant à partir d'une ouverture d'alimentation proximale (17) de la région tubulaire (13), à travers la région tubulaire et jusqu'à l'au moins une ouverture (19), et le ballon (14) étant relié à un canal de remplissage (20) s'étendant le long de la région tubulaire (13) jusqu'à l'extrémité proximale et présentant une ouverture de remplissage (21) avec un moyen de fermeture (23), le moyen de fermeture (23) présentant un élément de fermeture amovible fermant la section transversale de l'ouverture de remplissage (21), l'élément de fermeture ainsi que la sonde PEG présentant à l'extrémité proximale des moyens permettant de verrouiller l'élément de fermeture sur la sonde PEG, et l'élément de fermeture présentant des moyens pour l'application d'une clé permettant de supprimer le verrouillage de l'élément de fermeture avec la sonde PEG (12).

16. Kit selon la revendication 15, dans lequel l'élément de fermeture est une tige filetée apte à être vissée dans un filetage intérieur dans l'ouverture de remplissage (21), et/ou dans lequel les moyens pour l'application d'une clé sont une prise d'outil et la clé est un outil susceptible d'être appliqué sur la prise d'outil.

17. Kit selon l'une des revendications 15 à 16, dans lequel le ballon (14) et/ou la sonde PEG (12) est constitué d'un matériau souple ou élastiquement déformable.

18. Kit selon l'une des revendications 1 à 17, comprenant une sonde PEG (12) avec une extension distale (14) insérable dans l'estomac, laquelle présente au moins une ouverture distale (19) suivie d'une région tubulaire proximale (13), un canal d'alimentation (18) s'étendant à partir d'une ouverture d'alimentation proximale de la région tubulaire, à travers la région tubulaire et jusqu'à l'au moins une ouverture (19), la sonde PEG (12) présentant un marquage (33, 34) à une distance de l'extension distale et/ou de l'extrémité proximale, pour indiquer si la sonde PEG (12) est insérée suffisamment profondément dans la douille de trocart.

19. Kit selon la revendication 18, dans lequel la sonde PEG (12) présente une extension distale sous la forme d'un ballon (14).

20. Kit selon la revendication 1 à 19, comprenant une sonde PEG (12) avec une extension distale insérable dans l'estomac sous la forme d'un ballon élastique (14) avec une douille d'insertion (39) entourant le ballon, dans laquelle le ballon (14) est disposé radialement dans un état comprimé de façon élastique, la douille d'insertion (39) avec la sonde PEG (12) disposée dans celle-ci pouvant être insérée dans la douille de trocart (5), et la sonde PEG (12) pouvant être glissée ensemble avec la ballon (14) dans une direction distale hors de la douille d'insertion (39) et hors de la douille de trocart, de manière à gonfler le ballon (14).

21. Kit selon la revendication 20, dans lequel le ballon (14) est rempli avec une mousse élastique, et/ou dans lequel la douille d'insertion (39) est une douille apte à être séparée dans le sens longitudinal, afin de retirer la douille d'insertion (39) hors de la douille de trocart (5) après la sortie du ballon (14).

22. Kit selon l'une des revendications 1 à 21, dans lequel le trocart présente un canal de guidage s'étendant dans le sens longitudinal pour un fil de guidage.
